(19) Europäisches Patentamt
European Patent Office
Office européen des brevets

(11) Veröffentlichungsnummer : **0 019 858**
**B1**

(12) # EUROPÄISCHE PATENTSCHRIFT

(45) Veröffentlichungstag der Patentschrift : **22.06.83**

(21) Anmeldenummer : **80102850.7**

(22) Anmeldetag : **22.05.80**

(51) Int. Cl.$^3$ : **C 07 C131/00**, C 07 D307/68, C 07 D249/08, C 07 D309/12, A 01 N 37/36, A 01 N 43/08, A 01 N 43/64, A 01 N 43/16// C07C103/30

(54) **N-Oximinoalkyl-anilide, Verfahren zu ihrer Herstellung sowie ihre Verwendung als Fungizide.**

(30) Priorität : **05.06.79 DE 2922759**

(43) Veröffentlichungstag der Anmeldung : **10.12.80 Patentblatt 80/25**

(45) Bekanntmachung des Hinweises auf die Patenterteilung : **22.06.83 Patentblatt 83/25**

(84) Benannte Vertragsstaaten : **AT BE CH DE FR GB IT LI NL SE**

(56) Entgegenhaltungen : **GB A 2 019 404**

**Die Akte enthält technische Angaben, die nach dem Eingang der Anmeldung eingereicht wurden und die nicht in dieser Patentschrift enthalten sind.**

(73) Patentinhaber : **BAYER AG**
**Zentralbereich Patente, Marken und Lizenzen**
**D-5090 Leverkusen 1, Bayerwerk (DE)**

(72) Erfinder : **Stetter, Jörg, Dr.**
**Pahlkestrasse 3**
**D-5600 Wuppertal-1 (DE)**
Erfinder : **Brandes, Wilhelm, Dr.**
**Eichendorffstrasse 3**
**D-5653 Leichlingen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 019 858 B1

N-Oximinoalkyl-anilide, Verfahren zu ihrer Herstellung sowie ihre Verwendung als Fungizide

Die vorliegende Erfindung betrifft neue N-Oximinoalkyl-anilide, mehrere Verfahren zu ihrer Herstellung und ihre Verwendung als Fungizide.

Es ist bereits bekannt geworden, daß Halogenacetanilide, wie beispielsweise N-Chloracetyl-N-(2-ethyl-6-methyl-phenyl)-alaninmethylester, mit gutem Erfolg zur Bekämpfung von pilzlichen Pflanzenkrankheiten eingesetzt werden können (vergleiche DE-OS 23 50 944). Deren Wirkung ist jedoch, insbesondere bei niedrigen Aufwandmengen und -konzentrationen, insbesondere auch bei der Bekämpfung von Phytophthora-Arten, nicht immer ganz befriedigend. Weiterhin ist das N-Dichloracetyl-N-(2-hydroximino-prop-1-yl)-anilin als Zwischenprodukt bekannt (vergleiche J.C.S. Chem. Comm. 1978, Seite 124).

Es wurden neue N-Oximinoalkyl-anilide der allgemeinen Formel

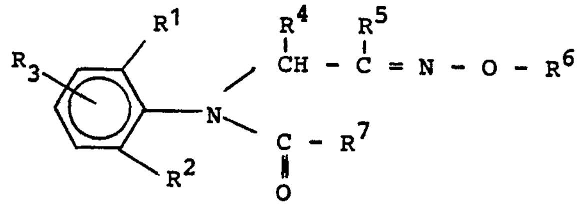

(I)

in welcher

$R^1$ für Wasserstoff, Alkyl oder Halogen steht,

$R^2$ für Wasserstoff oder Alkyl steht,

$R^3$ für Wasserstoff oder Alkyl steht,

$R^4$ für Wasserstoff oder Alkyl steht,

$R^5$ für Wasserstoff oder Alkyl steht,

$R^6$ für Wasserstoff, Alkyl, Alkenyl, Alkinyl, Alkoxyalkyl, Alkylthioalkyl oder gegebenenfalls durch Halogen, Alkyl, Halogenalkyl, Alkoxy, Alkylthio, Cyano und Nitro substituiertes Aralkyl steht und

$R^7$ für Furyl, Tetrahydrofuryl, Thiophenyl, Tetrahydrothiophenyl ; gegebenenfalls durch Alkyl substituiertes Isoxazolyl ; gegebenenfalls durch Cyano oder Thiocyano substituiertes Alkyl, Alkenyl und Alkinyl ; Dihalogenalkyl, Cycloalkyl ; sowie die Gruppierungen $—CH_2Az$, $—CH_2—OR^8$, $—CH_2—SR^8$, $—OR^8$, $—SR^8$, $—CH_2—OSO_2R^8$, $—COOR^8$ und

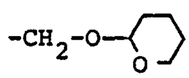

steht, wobei

$R^8$ für gegebenenfalls durch Halogen, Cyano und Thiocyano substituiertes Alkyl, Alkenyl, Alkinyl und Alkoxyalkyl steht,

und

Az für Pyrazol-1-yl, 1,2,4-Triazol-1-yl und Imidazol-1-yl steht, ausgenommen die Verbindung, in der

$R^1$ bis $R^4$ und $R^6$ für Wasserstoff,

$R^5$ für Methyl und

$R^7$ für Dichlormethyl stehen,

gefunden.

Die Verbindungen der Formel (I) können in der syn- oder anti-Form vorliegen ; vorwiegend fallen sie als Gemische beider Formen an.

Weiterhin wurde gefunden, daß man die N-Oximinoalkylanilide der Formel (I) erhält, wenn man

a) Anilinoalkyloxim-ether der Formel

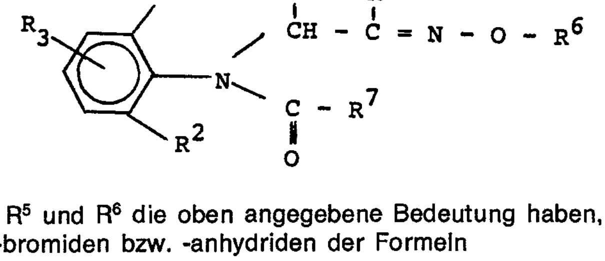

(II)

in welcher $R^1$, $R^2$, $R^3$, $R^4$, $R^5$ und $R^6$ die oben angegebene Bedeutung haben,

mit Säurechloriden oder -bromiden bzw. -anhydriden der Formeln

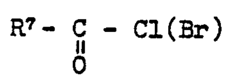

(IIIa)

bzw.

$$(R^7-\underset{\underset{O}{\|}}{C}-)_2O \qquad \text{(IIIb)}$$

in welchen $R^7$ die oben angegebene Bedeutung hat, in Gegenwart eines Verdünnungsmittels und gegebenenfalls in Gegenwart eines Säurebindemittels umsetzt, oder

b) Anilide der Formel

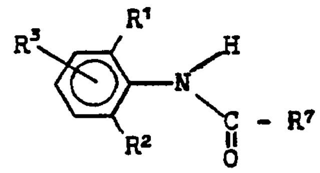

(IV)

in welcher $R^1$, $R^2$, $R^3$ und $R^7$ die oben angegebene Bedeutung haben, mit substituierten Oxim-ethern der Formel

$$Y-\overset{R^4}{\overset{|}{C}H}-\overset{R^5}{\overset{|}{C}}=N-O-R^6 \qquad \text{(V)}$$

in welcher

$R^4$, $R^5$ und $R^6$ die oben angegebene Bedeutung haben und

Y für Halogen, den Mesylat-oder Tosylat-Rest steht,

in Gegenwart eines Säurebinders und gegebenenfalls in Gegenwart eines Verdünnungsmittels umsetzt, oder

c) N-substituierte Anilide der Formel

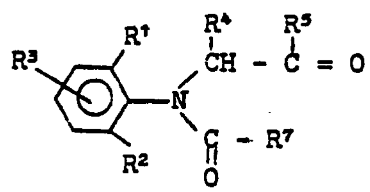

(VI)

in welcher $R^1$, $R^2$, $R^3$, $R^4$, $R^5$ und $R^7$ die oben angegebene Bedeutung haben, mit Salzen von Hydroxylamin-(Derivaten) der Formel

$$H_2N-O-R^6 \qquad \text{(VII)}$$

in welcher $R^6$ die oben angegebene Bedeutung hat, in Gegenwart eines Verdünnungsmittels und in Gegenwart eines Säurebindemittels umsetzt, oder

d) Alkalisalze von Oximen der Formel

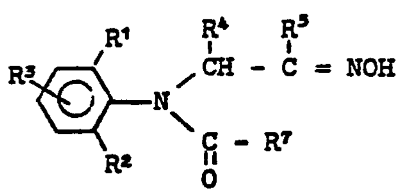

(VIII)

in welcher $R^1$, $R^2$, $R^3$, $R^4$, $R^5$ und $R^7$ die oben angegebene Bedeutung haben, mit Verbindungen der Formel

$$X-R^9 \qquad \text{(IX)}$$

in welcher

$R^9$ für Alkyl, Alkenyl, Alkinyl, Alkoxy-alkyl, Alkylthioalkyl oder gegebenenfalls substituiertes Aralkyl steht und

X für Chlor, Brom, Mesyl, Tosyl oder Methoxysulfonyloxy steht,

in Gegenwart eines organischen Verdünnungsmittels oder in Gegenwart eines organisch-wässrigen Zweiphasensystems in Gegenwart eines Phasentransferkatalysators umsetzt, wobei die Alkalisalze der

Oxime der Formel (VIII) in situ erzeugt werden, oder

e) Halogenacetanilide der Formel

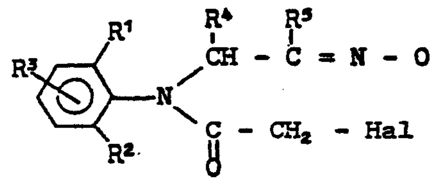

(X)

in welcher

$R^1$, $R^2$, $R^3$, $R^4$, $R^5$ und $R^6$ die oben angegebene Bedeutung haben und

Hal für Chlor, Brom oder Jod steht,

mit Verbindungen der Formel

$$B—R^{10} \quad (XI)$$

in welcher

B für Wasserstoff oder ein Alkalimetall steht und

$R^{10}$ für Az und die Gruppierungen $—OR^8$ oder $—SR^8$ steht,

in Gegenwart eines Verdünnungsmittels und gegebenenfalls in Gegenwart eines Säurebinders umsetzt, oder

f) Hydroxyacetanilide der Formel

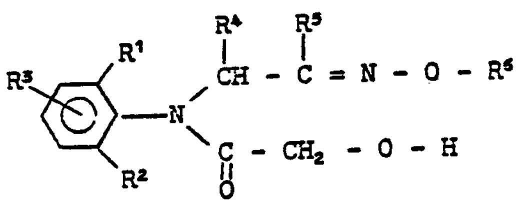

(XII)

in welcher $R^1$, $R^2$, $R^3$, $R^4$, $R^5$, und $R^6$ die oben angegebene Bedeutung haben,

(1) gegebenenfalls nach Aktivierung mittels Alkalimetall mit Halogeniden der Formel

$$Hal—R^{11} \quad (XIII)$$

in welcher

Hal die oben angegebene Bedeutung hat und

$R^{11}$ für den Rest $R^8$ sowie die Gruppierung $—SO_2R^8$ steht, wobei

$R^8$ die oben angegebene Bedeutung hat,

in Gegenwart eines Verdünnungsmittels und gegebenenfalls in Gegenwart eines Säurebinders umsetzt, oder

(2) mit Dihydropyran der Formel

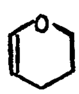

(XIV)

in Gegenwart eines Verdünnungsmittels und gegebenenfalls in Gegenwart eines Katalysators umsetzt.

Die neuen N-Oximinoalkyl-anilide weisen starke fungizide Eigenschaften auf. Dabei zeigen überraschenderweise die erfindungsgemäßen Verbindungen eine erheblich höhere Wirkung als der aus dem Stand der Technik bekannte N-Chloracetyl-N-(2-ethyl-6-methylphenyl)-alaninester, welcher chemisch und wirkungsmäßig eine naheliegende Verbindung ist. Die erfindungsgemäßen Stoffe stellen somit eine Bereicherung der Technik dar.

Die erfindungsgemäßen N-Oximinoalkyl-anilide sind durch die Formel (I) allgemein definiert. In dieser Formel stehen $R^1$, $R^2$, $R^3$, $R^4$ und $R^5$ vorzugsweise für Wasserstoff und geradkettiges oder verzweigtes Alkyl mit 1 bis 4 Kohlenstoffatomen. $R^1$ steht außerdem vorzugsweise für Halogen, wie insbesondere Fluor, Chlor oder Brom. $R^6$ steht vorzugsweise für Wasserstoff, geradkettiges oder verzweigtes Alkyl mit 1 bis 4 Kohlenstoffatomen, Alkenyl und Alkinyl mit jeweils 2 bis 4 Kohlenstoffatomen, Alkoxyalkyl und Alkylthioalkyl mit jeweils 1 bis 4 Kohlenstoffatomen im Alkylteil und 1 bis 4 Kohlenstoffatomen im Alkoxy bzw. Alkylthio-Teil, sowie für gegebenenfalls substituiertes Aralkyl mit 6 bis 10 Kohlenstoffatomen im Arylteil und 1 bis 2 Kohlenstoffatomen im Alkylteil, wie insbesondere Benzyl, wobei als Substituenten vorzugsweise infrage kommen ; Halogen, geradkettiges oder verzweigtes Alkyl mit 1 bis 4 Kohlenstoffatomen. Halogenalkyl mit 1 bis 2 Kohlenstoff- und 1 bis 5 Halogenatomen, wobei

als Halogene vorzugsweise Fluor und Chlor genannt seien, Alkoxy und Alkylthio mit jeweils 1 bis 2 Kohlenstoffatomen, sowie Cyano und Nitro.

$R^7$ steht vorzugsweise für Furyl, Tetrahydrofuryl, Thiophenyl, Tetrahydrothiophenyl, für gegebenenfalls durch Methyl oder Ethyl substituiertes Isoxazolyl sowie für gegebenenfalls durch Cyano oder Thiocyano substituiertes Alkyl mit 1 bis 4 und Alkenyl sowie Alkinyl mit jeweils 2 bis 4 Kohlenstoffatomen; ferner vorzugsweise für Dihalogenalkyl mit 1 bis 2 Kohlenstoffatomen, wobei als Halogenatome vorzugsweise Fluor und Chlor genannt seien und Cycloalkyl mit 3 bis 7 Kohlenstoffatomen, sowie für die Gruppierungen $-CH_2-Az$, $-CH_2-OR^8$, $-CH_2-SR^8$, $-OR^8$, $-SR^8$, $-CH_2-OSO_2R^8$, $-COOR^8$ und

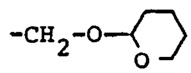

Az steht dabei vorzugsweise für Pyrazol-1-yl, 1,2,4-Triazol-1-yl und Imidazol-1-yl. $R^8$ steht vorzugsweise für gegebenenfalls durch Halogen, insbesondere Fluor, Chlor und Brom, Cyano und Thiocyano substituiertes Alkyl mit 1 bis 4 und Alkenyl sowie Alkinyl mit jeweils 2 bis 4 Kohlenstoffatomen und für Alkoxyalkyl mit 1 bis 4 Kohlenstoffatomen in jedem Alkylteil, ausgenommen die Verbindung in der

$R^1$ bis $R^4$ und $R^6$ für Wasserstoff,
$R^5$ für Methyl und
$R^7$ für Dichlormethyl stehen.

Ganz besonders bevorzugt sind diejenigen substituierten N-Oximino-alkyl-anilide der Formel (I), in denen $R^1$, $R^2$ und $R^3$ für Wasserstoff, Methyl, Ethyl, Isopropyl, sek.-Butyl und tert.-Butyl stehen; $R^1$ außerdem für Chlor oder Brom steht; $R^4$ für Wasserstoff, Methyl oder Ethyl steht; $R^5$ für Wasserstoff, Methyl oder Ethyl steht; $R^6$ für Wasserstoff, Methyl, Ethyl, Isopropyl, n-Propyl, n-Butyl, sek.-Butyl, tert.-Butyl, Vinyl, Allyl, Propargyl, Methoxymethyl, Ethoxymethyl, Ethoxyethyl, Methylthiomethyl und gegebenenfalls durch Chlor und/oder Methyl und/oder Methoxy und/oder Methylthio und/oder Trifluormethyl substituiertes Benzyl steht; und $R^7$ für 2-Furyl, 2-Thienyl, 2-Tetrahydrofuryl, 5-Methylisoxazol-3-yl, Methoxymethyl, Ethoxymethyl, Allyloxymethyl, Propargyloxymethyl, Ethoxymethoxymethyl, Methylmercaptomethyl, Methoxy, Ethoxy, Methylmercapto, Methylsulfonyloxymethyl, Methoxycarbonyl, Ethoxycarbonyl, Dichlormethyl, Cyclopropyl, Cyclohexyl, Pyrazol-1-yl-methyl, Imidazol-1-yl-methyl, 1,2,4-Triazol-1-yl-methyl und Tetrahydropyran-2-yl-oxymethyl steht, ausgenommen die Verbindungen, in der

$R^1$ bis $R^4$ und $R^6$ für Wasserstoff,
$R^5$ für Methyl und
$R^7$ für Dichlormethyl stehen.

Im einzelnen seien außer den bei den Herstellungsbeispielen genannten Verbindungen die folgenden Verbindungen der allgemeinen Formel (I) genannt:

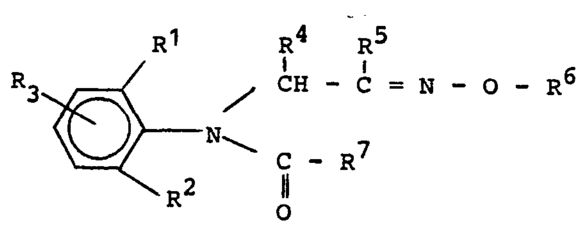

(I)

| $R^1$ | $R^2$ | $R^3$ | $R^4$ | $R^5$ | $R^6$ | $R^7$ |
|---|---|---|---|---|---|---|
| $CH_3$ | $CH_3$ | H | H | H | H | $-CH_2OCH_3$ |
| $CH_3$ | $CH_3$ | H | H | H | H | 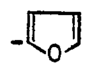 |
| $CH_3$ | $CH_3$ | H | H | H | H | $-CHCl_2$ |
| $CH_3$ | $CH_3$ | H | H | H | H | $-CO-O-CH_3$ |
| $CH_3$ | $CH_3$ | H | H | H | H | 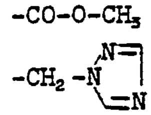 |
| $CH_3$ | $CH_3$ | H | H | H | H | 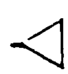 |
| $CH_3$ | $CH_3$ | H | H | H | H | 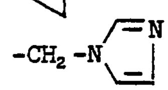 |
| $CH_3$ | $CH_3$ | H | H | H | $CH_3$ | 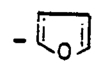 |

| $R^1$ | $R^2$ | $R^3$ | $R^4$ | $R^5$ | $R^6$ | $R^7$ |
|---|---|---|---|---|---|---|
| $CH_3$ | $CH_3$ | H | H | H | $CH_3$ | $-CHCl_2$ |
| $CH_3$ | $CH_3$ | H | H | H | $CH_3$ | $-CO-O-CH_3$ |
| $CH_3$ | $CH_3$ | H | H | H | $CH_3$ | $-CH_2-$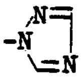 |
| $CH_3$ | $CH_3$ | H | H | H | $CH_3$ | 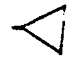 |
| $CH_3$ | $CH_3$ | H | H | H | $CH_3$ | $-CH_2-$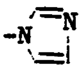 |
| $CH_3$ | $CH_3$ | H | H | H | $C_2H_5$ | - 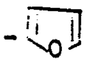 |
| $CH_3$ | $CH_3$ | H | H | H | $C_2H_5$ | $-CHCl_2$ |
| $CH_3$ | $CH_3$ | H | H | H | $C_2H_5$ | $-CO-O-CH_3$ |
| $CH_3$ | $CH_3$ | H | H | H | $C_2H_5$ | $-CH_2-$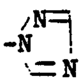 |
| $CH_3$ | $CH_3$ | H | H | H | $C_2H_5$ | 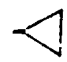 |
| $CH_3$ | $CH_3$ | H | H | H | $C_2H_5$ | $-CH_2-$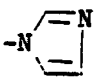 |
| $CH_3$ | $CH_3$ | H | H | H | $n-C_3H_7$ | - 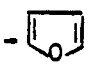 |
| $CH_3$ | $CH_3$ | H | H | H | $n-C_3H_7$ | $-CHCl_2$ |
| $CH_3$ | $CH_3$ | H | H | H | $n-C_3H_7$ | $-CO-O-CH_3$ |
| $CH_3$ | $CH_3$ | H | H | H | $n-C_3H_7$ | $-CH_2-$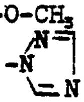 |
| $CH_3$ | $CH_3$ | H | H | H | $n-C_3H_7$ | 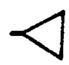 |
| $CH_3$ | $CH_3$ | H | H | H | $n-C_3H_7$ | $-CH_2-$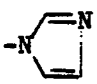 |
| $CH_3$ | $CH_3$ | H | H | H | $n-C_4H_9$ | $-CH_2OCH_3$ |
| $CH_3$ | $CH_3$ | H | H | H | $n-C_4H_9$ | - 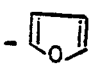 |
| $CH_3$ | $CH_3$ | H | H | H | $n-C_4H_9$ | $-CHCl_2$ |
| $CH_3$ | $CH_3$ | H | H | H | $n-C_4H_9$ | $-CO-O-CH_3$ |
| $CH_3$ | $CH_3$ | H | H | H | $n-C_4H_9$ | $-CH_2-$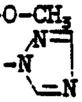 |
| $CH_3$ | $CH_3$ | H | H | H | $n-C_4H_9$ | 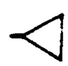 |
| $CH_3$ | $CH_3$ | H | H | H | $n-C_4H_9$ | $-CH_2-$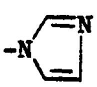 |
| $CH_3$ | $CH_3$ | H | H | $CH_3$ | H | $-CHCl_2$ |
| $CH_3$ | $CH_3$ | H | H | $CH_3$ | H | $-CO-O-CH_3$ |
| $CH_3$ | $CH_3$ | H | H | $CH_3$ | H | $-CH_2-$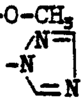 |
| $CH_3$ | $CH_3$ | H | H | $CH_3$ | H | 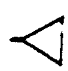 |
| $CH_3$ | $CH_3$ | H | H | $CH_3$ | H | $-CH_2-$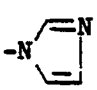 |

| $R^1$ | $R^2$ | $R^3$ | $R^4$ | $R^5$ | $R^6$ | $R^7$ |
|---|---|---|---|---|---|---|
| $CH_3$ | $CH_3$ | H | H | $CH_3$ | $CH_3$ | - 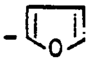 |
| $CH_3$ | $CH_3$ | H | H | $CH_3$ | $CH_3$ | $-CHCl_2$ |
| $CH_3$ | $CH_3$ | H | H | $CH_3$ | $CH_3$ | $-CO-O-CH_3$ |
| $CH_3$ | $CH_3$ | H | H | $CH_3$ | $CH_3$ | $-CH_2-$ 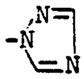 |
| $CH_3$ | $CH_3$ | H | H | $CH_3$ | $CH_3$ | 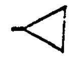 |
| $CH_3$ | $CH_3$ | H | H | $CH_3$ | $CH_3$ | $-CH_2-$ 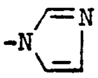 |
| $CH_3$ | $CH_3$ | H | H | $CH_3$ | $C_2H_5$ | $-CH_2OCH_2$ |
| $CH_3$ | $CH_3$ | H | H | $CH_3$ | $C_2H_5$ | - 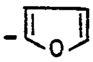 |
| $CH_3$ | $CH_3$ | H | H | $CH_3$ | $C_2H_5$ | $-CHCl_2$ |
| $CH_3$ | $CH_3$ | H | H | $CH_3$ | $C_2H_5$ | $-CO-O-CH_3$ |
| $CH_3$ | $CH_3$ | H | H | $CH_3$ | $C_2H_5$ | $-CH_2-$ 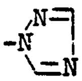 |
| $CH_3$ | $CH_3$ | H | H | $CH_3$ | $C_2H_5$ | 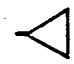 |
| $CH_3$ | $CH_3$ | H | H | $CH_3$ | $C_2H_5$ | $-CH_2-$ 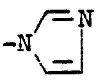 |
| $CH_3$ | $CH_3$ | H | H | $CH_3$ | $n-C_3H_7$ | $-CH_2OCH_3$ |
| $CH_3$ | $CH_3$ | H | H | $CH_3$ | $n-C_3H_7$ | - 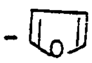 |
| $CH_3$ | $CH_3$ | H | H | $CH_3$ | $n-C_3H_7$ | $-CHCl_2$ |
| $CH_3$ | $CH_3$ | H | H | $CH_3$ | $n-C_3H_7$ | $-CO-O-CH_3$ |
| $CH_3$ | $CH_3$ | H | H | $CH_3$ | $n-C_3H_7$ | $-CH_2-$ 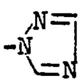 |
| $CH_3$ | $CH_3$ | H | H | $CH_3$ | $n-C_3H_7$ | 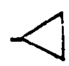 |
| $CH_3$ | $CH_3$ | H | H | $CH_3$ | $n-C_3H_7$ | $-CH_2-$ 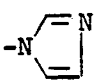 |
| $CH_3$ | $CH_3$ | H | H | $CH_3$ | $n-C_4H_9$ | $-CH_2OCH_3$ |
| $CH_3$ | $CH_3$ | H | H | $CH_3$ | $n-C_4H_9$ | - 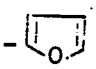 |
| $CH_3$ | $CH_3$ | H | H | $CH_3$ | $n-C_4H_9$ | $-CHCl_2$ |
| $CH_3$ | $CH_3$ | H | H | $CH_3$ | $n-C_4H_9$ | $-CO-O-CH_3$ |
| $CH_3$ | $CH_3$ | H | H | $CH_3$ | $n-C_4H_9$ | $-CH_2-$ 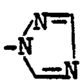 |
| $CH_3$ | $CH_3$ | H | H | $CH_3$ | $n-C_4H_9$ | 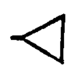 |
| $CH_3$ | $CH_3$ | H | H | $CH_3$ | $n-C_4H_9$ | $-CH_2-$ 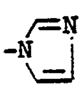 |
| $CH_3$ | $CH_3$ | H | $CH_3$ | H | H | $-CH_2OCH_3$ |
| $CH_3$ | $CH_3$ | H | $CH_3$ | H | H | - 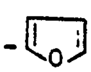 |

| $R^1$ | $R^2$ | $R^3$ | $R^4$ | $R^5$ | $R^6$ | $R^7$ |
|---|---|---|---|---|---|---|
| $CH_3$ | $CH_3$ | H | $CH_3$ | H | H | $-CHCl_2$ |
| $CH_3$ | $CH_3$ | H | $CH_3$ | H | H | $-CO-O-CH_3$ |
| $CH_3$ | $CH_3$ | H | $CH_3$ | H | H | $-CH_2-$ 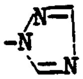 |
| $CH_3$ | $CH_3$ | H | $CH_3$ | H | H | 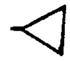 |
| $CH_3$ | $CH_3$ | H | $CH_3$ | H | H | $-CH_2-$ 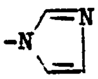 |
| $CH_3$ | $CH_3$ | H | $CH_3$ | H | $CH_3$ | $-CH_2-$ 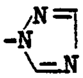 |
| $CH_3$ | $CH_3$ | H | $CH_3$ | H | $CH_3$ | 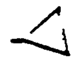 |
| $CH_3$ | $CH_3$ | H | $CH_3$ | H | $C_2H_5$ | - 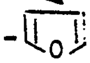 |
| $CH_3$ | $CH_3$ | H | $CH_3$ | H | $C_2H_5$ | $-CH_2-$ 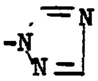 |
| $CH_3$ | $CH_3$ | H | $CH_3$ | H | $C_2H_5$ | 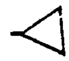 |
| $CH_3$ | $CH_3$ | H | $CH_3$ | H | $n-C_3H_7$ | $-CH_2OCH_3$ |
| $CH_3$ | $CH_3$ | H | $CH_3$ | H | $n-C_3H_7$ | - 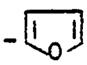 |
| $CH_3$ | $CH_3$ | H | $CH_3$ | H | $n-C_3H_7$ | $-CHCl_2$ |
| $CH_3$ | $CH_3$ | H | $CH_3$ | H | $n-C_3H_7$ | $-CO-O-CH_3$ |
| $CH_3$ | $CH_3$ | H | $CH_3$ | H | $n-C_3H_7$ | $-CH_2-$ 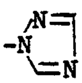 |
| $CH_3$ | $CH_3$ | H | $CH_3$ | H | $n-C_3H_7$ | 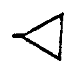 |
| $CH_3$ | $CH_3$ | H | $CH_3$ | H | $n-C_3H_7$ | $-CH_2-$ 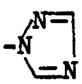 |
| $CH_3$ | $CH_3$ | H | $CH_3$ | H | $n-C_4H_9$ | $-CH_2OCH_3$ |
| $CH_3$ | $CH_3$ | H | $CH_3$ | H | $n-C_4H_9$ | - 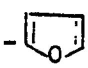 |
| $CH_3$ | $CH_3$ | H | $CH_3$ | H | $n-C_4H_9$ | $-CHCl_2$ |
| $CH_3$ | $CH_3$ | H | $CH_3$ | H | $n-C_4H_9$ | $-CO-O-CH_3$ |
| $CH_3$ | $CH_3$ | H | $CH_3$ | H | $n-C_4H_9$ | $-CH_2-$ 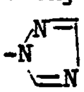 |
| $CH_3$ | $CH_3$ | H | $CH_3$ | H | $n-C_4H_9$ | 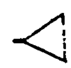 |
| $CH_3$ | $CH_3$ | H | $CH_3$ | H | $n-C_4H_9$ | $-CH_2-$ 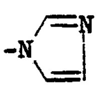 |
| $CH_3$ | $CH_3$ | H | $CH_3$ | $CH_3$ | H | $-CH_2OCH_3$ |
| $CH_3$ | $CH_3$ | H | $CH_3$ | $CH_3$ | H | - 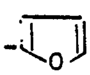 |
| $CH_3$ | $CH_3$ | H | $CH_3$ | $CH_3$ | H | $-CHCl_2$ |
| $CH_3$ | $CH_3$ | H | $CH_3$ | $CH_3$ | H | $-CO-O-CH_3$ |
| $CH_3$ | $CH_3$ | H | $CH_3$ | $CH_3$ | H | $-CH_2-$ 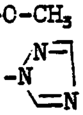 |

| $R^1$ | $R^2$ | $R^3$ | $R^4$ | $R^5$ | $R^6$ | $R^7$ |
|---|---|---|---|---|---|---|
| $CH_3$ | $CH_3$ | H | $CH_3$ | $CH_3$ | H | 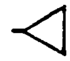 |
| $CH_3$ | $CH_3$ | H | $CH_3$ | $CH_3$ | H | $-CH_2-$ 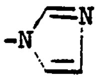 |
| $CH_3$ | $CH_3$ | H | $CH_3$ | $CH_3$ | $CH_3$ | 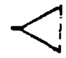 |
| $CH_3$ | $CH_3$ | H | $CH_3$ | $CH_3$ | $C_2H_5$ | $-CH_2OCH_3$ |
| $CH_3$ | $CH_3$ | H | $CH_3$ | $CH_3$ | $C_2H_5$ | - 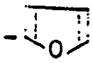 |
| $CH_3$ | $CH_3$ | H | $CH_3$ | $CH_3$ | $C_2H_5$ | $-CHCl_2$ |
| $CH_3$ | $CH_3$ | H | $CH_3$ | $CH_3$ | $C_2H_5$ | $-CO-O-CH_3$ |
| $CH_3$ | $CH_3$ | H | $CH_3$ | $CH_3$ | $C_2H_5$ | $-CH_2-$ 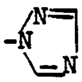 |
| $CH_3$ | $CH_3$ | H | $CH_3$ | $CH_3$ | $C_2H_5$ | 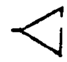 |
| $CH_3$ | $CH_3$ | H | $CH_3$ | $CH_3$ | $C_2H_5$ | $-CH_2-$ 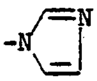 |
| $CH_3$ | $CH_3$ | H | $CH_3$ | $CH_3$ | $n-C_3H_7$ | $-CH_2OCH_3$ |
| $CH_3$ | $CH_3$ | H | $CH_3$ | $CH_3$ | $n-C_3H_7$ | - 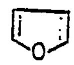 |
| $CH_3$ | $CH_3$ | H | $CH_3$ | $CH_3$ | $n-C_3H_7$ | $-CHCl_2$ |
| $CH_3$ | $CH_3$ | H | $CH_3$ | $CH_3$ | $n-C_3H_7$ | $-CO-O-CH_3$ |
| $CH_3$ | $CH_3$ | H | $CH_3$ | $CH_3$ | $n-C_3H_7$ | $-CH_2-O-$ 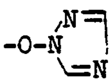 |
| $CH_3$ | $CH_3$ | H | $CH_3$ | $CH_3$ | $n-C_3H_7$ | 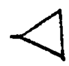 |
| $CH_3$ | $CH_3$ | H | $CH_3$ | $CH_3$ | $n-C_3H_7$ | $-CH_2-$ 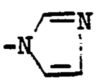 |
| $CH_3$ | $CH_3$ | H | $CH_3$ | $CH_3$ | $n-C_4H_9$ | $-CH_2OCH_3$ |
| $CH_3$ | $CH_3$ | H | $CH_3$ | $CH_3$ | $n-C_4H_9$ | - 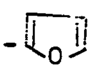 |
| $CH_3$ | $CH_3$ | H | $CH_3$ | $CH_3$ | $n-C_4H_9$ | $-CHCl_2$ |
| $CH_3$ | $CH_3$ | H | $CH_3$ | $CH_3$ | $n-C_4H_9$ | $-CO-O-CH_3$ |
| $CH_3$ | $CH_3$ | H | $CH_3$ | $CH_3$ | $n-C_4H_9$ | $-CH_2-$ 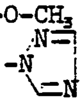 |
| $CH_3$ | $CH_3$ | H | $CH_3$ | $CH_3$ | $n-C_4H_9$ | 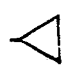 |
| $CH_3$ | $CH_3$ | H | $CH_3$ | $CH_3$ | $n-C_4H_9$ | $-CH_2-$ 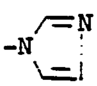 |
| $CH_3$ | $CH_3$ | H | $CH_3$ | $CH_3$ | $CH_3$ | $-CH_2-$ 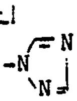 |
| $CH_3$ | $CH_3$ | H | $CH_3$ | $CH_3$ | $CH_3$ | $-COOCH_3$ |
| $C_2H_5$ | $C_2H_5$ | H | $CH_3$ | H | $CH_3$ | $-CH_2OCH_3$ |
| $CH_3$ | $CH_3$ | H | $CH_3$ | H | $CH_3$ | $-CH_2-$ 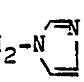 |
| $CH_3$ | $CH_3$ | H | H | $CH_3$ | $-CH_2-CH=CH_2$ | $-CH_2OCH_3$ |

| $R^1$ | $R^2$ | $R^3$ | $R^4$ | $R^5$ | $R^6$ | $R^7$ |
|---|---|---|---|---|---|---|
| $CH_3$ | $CH_3$ | H | H | $CH_3$ | $-CH_2-C{\equiv}CH$ | $-CH_2OCH_3$ |
| $CH_3$ | $CH_3$ | H | H | $CH_3$ | $-CH_2-CH=CH_2$ | 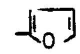 |
| $CH_3$ | $CH_3$ | H | H | $CH_3$ | 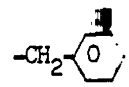 | $-CH_2OCH_3$ |
| $CH_3$ | $CH_3$ | H | $CH_3$ | $CH_3$ | $CH_3$ | 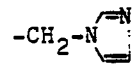 |
| Cl | $CH_3$ | H | H | $CH_3$ | $CH_3$ | $-CH_2OCH_3$ |
| Cl | $CH_3$ | H | H | $CH_3$ | $CH_3$ | 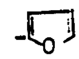 |
| $CH_3$ | $CH_3$ | H | H | H | $CH_3$ | $-CH_2OCH_2-C{\equiv}CH$ |
| $CH_3$ | $CH_3$ | H | H | H | $CH_3$ | $-CH_2OCH_2-CH=CH_2$ |
| Cl | $CH_3$ | H | $CH_3$ | H | $CH_3$ | $-CH_2OCH_3$ |

Verwendet man beispielsweise 2,6-Dimethylanilinomethyl-methylketoxim-methylether und Methoxyessigsäurechlorid als Ausgangsstoffe, so kann der Reaktionsablauf durch das folgende Formelschema wiedergegeben werden (Verfahren a) :

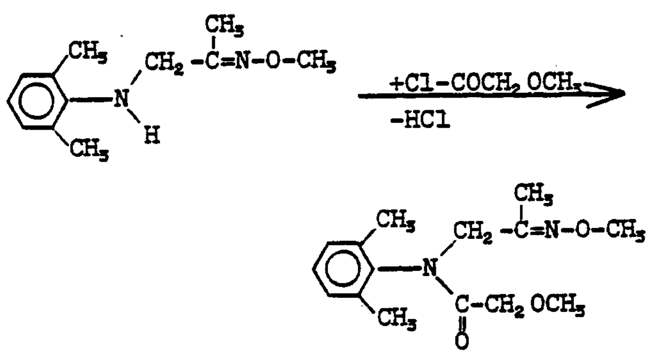


Verwendet man beispielsweise 2,6-Dimethyl-N-(2-furoyl)-anilin und Chlormethylaldoxim-methylether als Ausgangsstoffe, so kann der Reaktionsablauf durch das folgende Formelschema wiedergegeben werden (Verfahren b) :

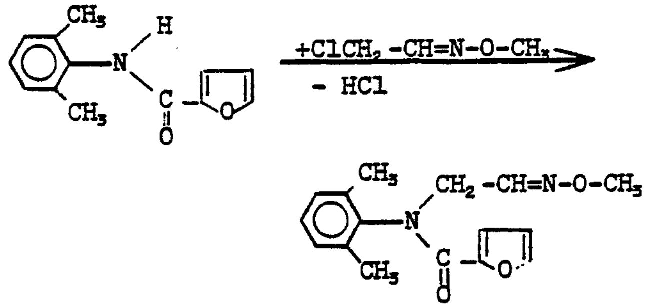


Verwendet man beispielsweise 2,6-Dimethyl-N-acetonyl-methoxy-acetanilid und O-Methyl-hydroxylamin als Ausgangsstoffe, so kann der Reaktionsablauf durch das folgende Formelschema wiedergegeben werden (Verfahren c) :

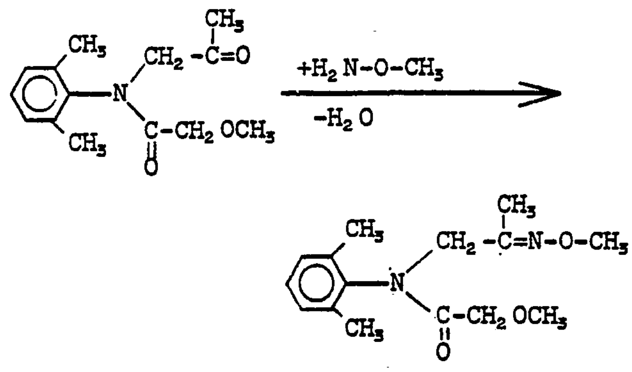


Verwendet man beispielsweise das Natriumsalz von 2,6-Dimethyl-methoxyacetanilido-methylmethylketoxim und Benzylchlorid als Ausgangsstoffe, so kann der Reaktionsablauf durch das folgende Formelschema wiedergegeben werden (Verfahren d) :

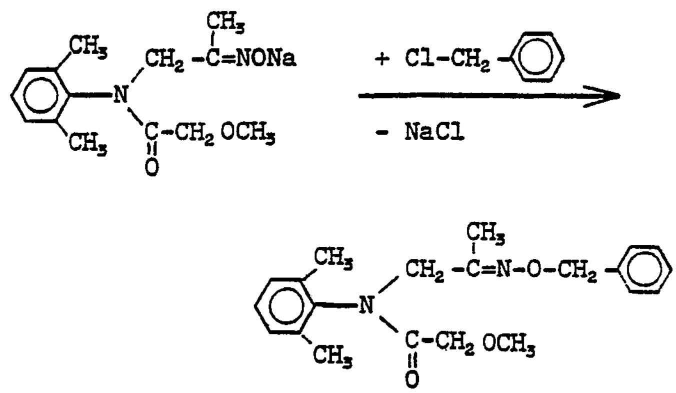


Verwendet man beispielsweise 2,6-Dimethyl-N-(2'-methoxyimino-ethyl)-chloracetanilid und 1,2,4-Triazol als Ausgangsstoffe, so kann der Reaktionsablauf durch das folgende Formelschema wiedergegeben werden (Verfahren e) :

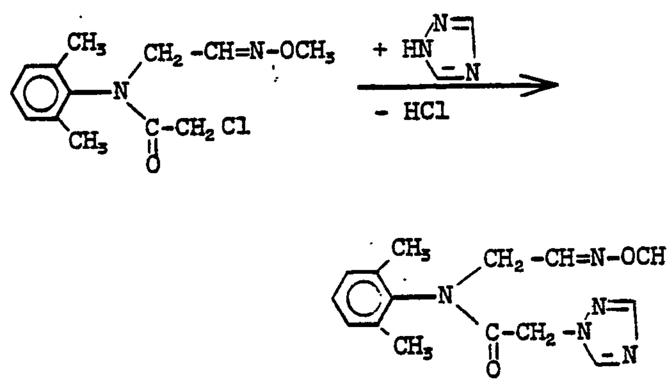


Verwendet man beispielsweise 2,6-Dimethyl-N-(2'-methoxyimino-ethyl)hydroxylacetanilid und Ethoxymethylchlorid als Ausgangsstoffe, so kann der Reaktionsablauf durch das folgende Formelschema wiedergegeben werden (Verfahren f/1) :

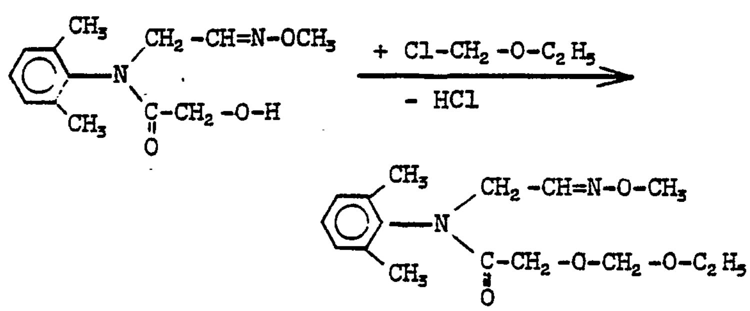

Verwendet man beispielsweise 2,6-Dimethyl-N-(2'-methoxyimino-ethyl)-hydroxy-acetanilid und 3,4-Dihydro-2H-pyran als Ausgangsstoffe, so kann der Reaktionsablauf durch das folgende Formelschema wiedergegeben werden (Verfahren f/2) :

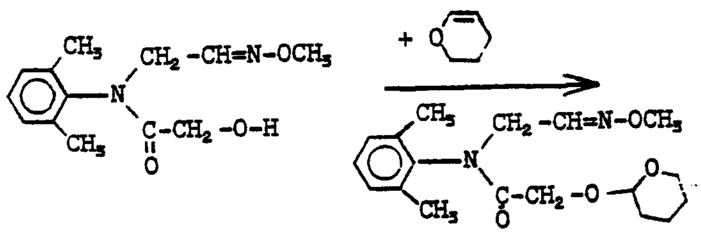

Die bei der Durchführung des erfindungsgemäßen Verfahrens (a) als Ausgangsstoffe benötigten Anilinoalkyl-oxim-ether sind durch die Formel (II) allgemein definiert. In dieser Formel stehen $R^1$, $R^2$, $R^3$, $R^4$, $R^5$ und $R^6$ vorzugsweise für diejenigen Reste, die bereits im Zusammenhang mit der Beschreibung der erfindungsgemäßen Stoffe der Formel (I) vorzugsweise für diese Reste genannt wurden.

Als Beispiele für Verbindungen der Formel (II) seien im einzelnen die folgenden Verbindungen genannt :

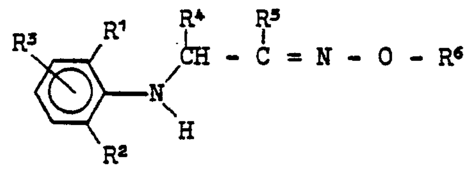

| $R^1$ | $R^2$ | $R^3$ | $R^4$ | $R^5$ | $R^6$ |
|---|---|---|---|---|---|
| H | H | H | H | H | H |
| $CH_3$ | $CH_3$ | H | H | H | H |
| $CH_3$ | $C_2H_5$ | H | H | H | H |
| $C_2H_5$ | $C_2H_5$ | H | H | H | H |
| $CH_3$ | $CH_3$ | H | H | H | $CH_3$ |
| $CH_3$ | $C_2H_5$ | H | H | H | $CH_3$ |
| $C_2H_5$ | $C_2H_5$ | H | H | H | $CH_3$ |
| $C_2H_5$ | H | H | H | H | $CH_3$ |
| $C(CH_3)_3$ | H | H | H | H | $CH_3$ |
| Cl | $CH_3$ | H | H | H | $CH_3$ |
| Br | $CH_3$ | H | H | H | $CH_3$ |
| Cl | $C(CH_3)_3$ | H | H | H | $CH_3$ |
| $CH_3$ | H | 3-$CH_3$ | H | H | $CH_3$ |
| i-$C_3H_7$ | $C_2H_5$ | H | H | H | $CH_3$ |
| $CH_3$ | H | H | H | H | $CH_3$ |
| $CH_3$ | $CH_3$ | H | H | H | $C_2H_5$ |
| $CH_3$ | $C_2H_5$ | H | H | H | $C_2H_5$ |
| $C_2H_5$ | $C_2H_5$ | H | H | H | $C_2H_5$ |
| $C_2H_5$ | H | H | H | H | $C_2H_5$ |
| $C(CH_3)_3$ | H | H | H | H | $C_2H_5$ |
| Cl | $CH_3$ | H | H | H | $C_2H_5$ |
| Br | $CH_3$ | H | H | H | $C_2H_5$ |
| Cl | $C(CH_3)_3$ | H | H | H | $C_2H_5$ |
| $CH_3$ | H | 3-$CH_3$ | H | H | $C_2H_5$ |
| i-$C_3H_7$ | $C_2H_5$ | H | H | H | $C_2H_5$ |
| $CH_3$ | H | H | H | H | $C_2H_5$ |

| $R^1$ | $R^2$ | $R^3$ | $R^4$ | $R^5$ | $R^6$ |
|---|---|---|---|---|---|
| $CH_3$ | $CH_3$ | H | H | H | n-$C_3H_7$ |
| $CH_3$ | $C_2H_5$ | H | H | H | n-$C_3H_7$ |
| $C_2H_5$ | $C_2H_5$ | H | H | H | n-$C_3H_7$ |
| $C(CH_3)_3$ | H | H | H | H | n-$C_3H_7$ |
| $CH_3$ | $CH_3$ | H | H | H | n-$C_4H_9$ |
| $CH_3$ | $C_2H_5$ | H | H | H | n-$C_4H_9$ |
| $C_2H_5$ | $C_2H_5$ | H | H | H | n-$C_4H_9$ |
| $C(CH_3)_3$ | H | H | H | H | n-$C_4H_9$ |
| $CH_3$ | $CH_3$ | H | $CH_3$ | H | $-CH_2-CH=CH_2$ |
| $CH_3$ | $C_2H_5$ | H | $CH_3$ | H | $-CH_2-CH=CH_2$ |
| $C_2H_5$ | $C_2H_5$ | H | $CH_3$ | H | $-CH_2-CH=CH_2$ |
| $C(CH_3)_3$ | H | H | $CH_3$ | H | $-CH_2-CH=CH_2$ |
| $CH_3$ | $CH_3$ | H | $CH_3$ | H | $-CH_2-C\equiv CH$ |
| $CH_3$ | $C_2H_5$ | H | $CH_3$ | H | $-CH_2-C\equiv CH$ |
| $C_2H_5$ | $C_2H_5$ | H | $CH_3$ | H | $-CH_2-C\equiv CH$ |
| $C(CH_3)_3$ | H | H | $CH_3$ | H | $-CH_2-C\equiv CH$ |
| $CH_3$ | $CH_3$ | H | $CH_3$ | H | $-CH_2-O-CH_3$ |
| $CH_3$ | $C_2H_5$ | H | $CH_3$ | H | $-CH_2-O-CH_3$ |
| $C_2H_5$ | $C_2H_5$ | H | $CH_3$ | H | $-CH_2-O-CH_3$ |
| $C(CH_3)_3$ | H | H | $CH_3$ | H | $-CH_2-O-CH_3$ |
| $CH_3$ | $CH_3$ | H | $CH_3$ | H | $-CH_2-$ 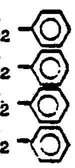 |
| $CH_3$ | $C_2H_5$ | H | $CH_3$ | H | $-CH_2-$ |
| $C_2H_5$ | $C_2H_5$ | H | $CH_3$ | H | $-CH_2-$ |
| $C(CH_3)_3$ | H | H | $CH_3$ | H | $-CH_2-$ |
| $CH_3$ | $CH_3$ | H | $CH_3$ | $CH_3$ | $CH_3$ |
| $CH_3$ | $C_2H_5$ | H | $CH_3$ | $CH_3$ | $CH_3$ |
| $C_2H_5$ | $C_2H_5$ | H | $CH_3$ | $CH_3$ | $CH_3$ |
| $C(CH_3)_3$ | H | H | $CH_3$ | $CH_3$ | $CH_3$ |
| $CH_3$ | $CH_3$ | H | $CH_3$ | $CH_3$ | $C_2H_5$ |
| $CH_3$ | $C_2H_5$ | H | $CH_3$ | $CH_3$ | $C_2H_5$ |
| $C_2H_5$ | $C_2H_5$ | H | $CH_3$ | $CH_3$ | $C_2H_5$ |
| $C(CH_3)_3$ | H | H | $CH_3$ | $CH_3$ | $C_2H_5$ |
| $CH_3$ | $CH_3$ | H | $CH_3$ | $CH_3$ | n-$C_3H_7$ |
| $CH_3$ | $C_2H_5$ | H | $CH_3$ | $CH_3$ | n-$C_3H_7$ |
| $C_2H_5$ | $C_2H_5$ | H | $CH_3$ | $CH_3$ | n-$C_3H_7$ |
| $C(CH_3)_3$ | H | H | $CH_3$ | $CH_3$ | n-$C_3H_7$ |
| $CH_3$ | $CH_3$ | H | $CH_3$ | $CH_3$ | n-$C_4H_9$ |
| $CH_3$ | $C_2H_5$ | H | $CH_3$ | $CH_3$ | n-$C_4H_9$ |
| $C_2H_5$ | $C_2H_5$ | H | $CH_3$ | $CH_3$ | n-$C_4H_9$ |
| $C(CH_3)_3$ | H | H | $CH_3$ | $CH_3$ | n-$C_4H_9$ |
| $CH_3$ | $CH_3$ | H | H | H | $CH_3$ |
| $CH_3$ | $C_2H_5$ | H | H | H | $CH_3$ |
| $C_2H_5$ | $C_2H_5$ | H | H | H | $CH_3$ |
| $C(CH_3)_3$ | H | H | H | H | $CH_3$ |
| $CH_3$ | $CH_3$ | H | H | H | $C_2H_5$ |
| $CH_3$ | $C_2H_5$ | H | H | H | $C_2H_5$ |
| $C_2H_5$ | $C_2H_5$ | H | H | H | $C_2H_5$ |
| $C(CH_3)_3$ | H | H | H | H | $C_2H_5$ |

| $R^1$ | $R^2$ | $R^3$ | $R^4$ | $R^5$ | $R^6$ |
|---|---|---|---|---|---|
| $CH_3$ | $CH_3$ | H | H | H | $n-C_3H_7$ |
| $CH_3$ | $C_2H_5$ | H | H | H | $n-C_3H_7$ |
| $C_2H_5$ | $C_2H_5$ | H | H | H | $n-C_3H_7$ |
| $C(CH_3)_3$ | H | H | H | H | $n-C_3H_7$ |
| $CH_3$ | $CH_3$ | H | H | H | $n-C_4H_9$ |
| $CH_3$ | $C_2H_5$ | H | H | H | $n-C_4H_9$ |
| $C_2H_5$ | $C_2H_5$ | H | H | H | $n-C_4H_9$ |
| $C(CH_3)_3$ | H | H | H | H | $n-C_4H_9$ |
| $CH_3$ | $CH_3$ | H | $CH_3$ | $CH_3$ | $-CH_2-CH=CH_2$ |
| $CH_3$ | $C_2H_5$ | H | $CH_3$ | $CH_3$ | $-CH_2-CH=CH_2$ |
| $C_2H_5$ | $C_2H_5$ | H | $CH_3$ | $CH_3$ | $-CH_2-CH=CH_2$ |
| $C(CH_3)_3$ | H | H | $CH_3$ | $CH_3$ | $-CH_2-CH=CH_2$ |
| $CH_3$ | $CH_3$ | H | $CH_3$ | $CH_3$ | $-CH_2-C\equiv CH$ |
| $CH_3$ | $C_2H_5$ | H | $CH_3$ | $CH_3$ | $-CH_2-C\equiv CH$ |
| $C_2H_5$ | $C_2H_5$ | H | $CH_3$ | $CH_3$ | $-CH_2-C\equiv CH$ |
| $C(CH_3)_3$ | H | H | $CH_3$ | $CH_3$ | $-CH_2-C\equiv CH$ |
| $CH_3$ | $CH_3$ | H | $CH_3$ | $CH_3$ | $-CH_2OCH_3$ |
| $CH_3$ | $C_2H_5$ | H | $CH_3$ | $CH_3$ | $-CH_2OCH_3$ |
| $C_2H_5$ | $C_2H_5$ | H | $CH_3$ | $CH_3$ | $-CH_3OCH_3$ |
| $C(CH_3)_3$ | H | H | $CH_3$ | $CH_3$ | $-CH_2OCH_3$ |
| $CH_3$ | $CH_3$ | H | $CH_3$ | $CH_3$ | $-CH_2-$ 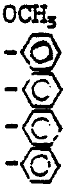 |
| $CH_3$ | $C_2H_5$ | H | $CH_3$ | $CH_3$ | $-CH_2-$ |
| $C_2H_5$ | $C_2H_5$ | H | $CH_3$ | $CH_3$ | $-CH_2-$ |
| $C(CH_3)_3$ | H | H | $CH_3$ | $CH_3$ | $-CH_2-$ |

Die Anilinoalkyloximether der Formel (II) sind noch nicht bekannt. Sie sind jedoch in einer eigenen, älteren Anmeldung beschrieben, die noch nicht bekannt ist (vergleiche die Deutsche Patentanmeldung P 28 47 827 [Le A 19 240] vom 3.11.1978). Danach können sie erhalten werden, indem man z. B. Aniline der Formel

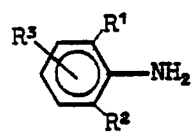

(XV)

in welcher $R^1$, $R^2$ und $R^3$ die oben angegebene Bedeutung haben, mit substituierten Oxim-ethern der Formel

$$Y-\underset{|}{\overset{R^4}{CH}}-\overset{R^5}{C}=N-O-R^6 \qquad (V)$$

in welcher $R^4$, $R^5$, $R^6$ und Y die oben angegebene Bedeutung haben, in Gegenwart eines Säurebinders, wie beispielsweise Alkalicarbonate, und gegebenenfalls in Gegenwart eines inerten organischen Lösungsmittels, wie beispielsweise Toluol oder Dimethylformamid, bei Temperaturen zwischen 20 und 160 °C umsetzt.

Die als Ausgangsstoffe benötigten Aniline der Formel (XV) sind allgemein bekannte Verbindungen der organischen Chemie. Als Beispiele seien genannt :

Anilin ; 2-Methylanilin ; 2-Ethylanilin ; 2-Isopropylanilin ; 2-sek.-Butylanilin ; 2-tert.-Butylanilin ; 2,6-Dimethylanilin ; 2,3-Dimethylanilin ; 2,5-Dimethylanilin ; 3,5-Dimethylanilin ; 2,6-Diethylanilin ; 2-Ethyl-6-methylanilin ; 2,3,4-Trimethylanilin ; 2,4,6-Trimethylanilin ; 2,4,5-Trimethylanilin ; 2-Ethyl-4,6-dimethylanilin ; 2,6-Diethyl-4-methylanilin ; 2,6-Diisopropyl-4-methylanilin ; 2,3,5-Trimethylanilin ; 2,3,6-Trimethylanilin ; 6-Chlor-2-methylanilin ; 2-Brom-6-methylanilin ; 2-Chlor-6-tert.-butylanilin.

Die außerdem als Ausgangsstoffe benötigten substituierten Oxim-ether der Formel (V) sind teilweise

bekannt (vergleiche US-Patentschrift 3 896 189 sowie J. Org. Chem. *36*, (1971) 3467) bzw. lassen sich nach den dort angegebenen Verfahren leicht herstellen (vergleiche entsprechende allgemeine Angaben beim Verfahren b).

Die außerdem für das erfindungsgemäße Verfahren (a) als Ausgangsstoffe zu verwendenden Säurechloride oder -bromide bzw. -anhydride sind durch die Formel (IIIa) und (IIIb) allgemein definiert. In diesen Formeln steht $R^7$ vorzugsweise für diejenigen Reste, die Bereits im Zusammenhang mit der Beschreibung der erfindungsgemäßen Stoffe der Formel (I) vorzugsweise für diesen Substituenten genannt wurden.

Die Säurechloride oder -bromide bzw. -anhydride der Formeln (IIIa) und (IIIb) sind allgemein bekannte Verbindungen der organischen Chemie.

Die bei der Durchführung des erfindungsgemäßen Verfahrens (b) als Ausgangsstoffe benötigten Anilide sind durch die Formel (IV) allgemein definiert. In dieser Formel stehen $R^1$, $R^2$, $R^3$ und $R^7$ vorzugsweise für diejenigen Reste, die bereits im Zusammenhang mit der Beschreibung der erfindungsgemäßen Stoffe der Formel (I) vorzugsweise für diese Substituenten genannt wurden.

Die Anilide der Formel (IV) können in allgemein bekannter Art und Weise erhalten werden, indem man entsprechende Aniline mit einem Säurechlorid oder -bromid bzw. -anhydrid der Formeln (IIIa) und (IIIb) gemäß den Bedingungen des Verfahrens (a) in Gegenwart eines inerten organischen Lösungsmittels, wie beispielsweise Toluol oder Methylenchlorid, gegebenenfalls in Gegenwart eines Säurebindemittels, wie z. B. Kaliumcarbonat oder Triethylamin, oder in Gegenwart eines Katalysators, wie z. B. Dimethylformamid, bei Temperaturen zwischen 0 und 100 °C umsetzt.

Als Beispiele für die Ausgangsstoffe der Formel (IV) seien genannt :

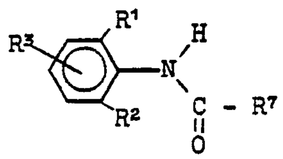

(IV)

| $R^1$ | $R^2$ | $R^3$ | $R^7$ |
|---|---|---|---|
| $CH_3$ | $CH_3$ | H | $-CH_2OCH_3$ |
| $CH_3$ | $C_2H_5$ | H | $-CH_2OCH_3$ |
| $C_2H_5$ | $C_2H_5$ | H | $-CH_2OCH_3$ |
| $CH_3$ | $CH_3$ | $3-CH_3$ | $-CH_2OCH_3$ |
| Cl | $CH_3$ | H | $-CH_2OCH_3$ |
| $CH_3$ | $CH_3$ | H | - 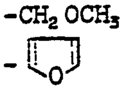 |
| $CH_3$ | $C_2H_5$ | H | - 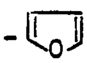 |
| $C_2H_5$ | $C_2H_5$ | H | - 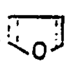 |
| $CH_3$ | $CH_3$ | $3-CH_3$ | - 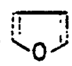 |
| Cl | $CH_3$ | H | - 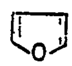 |
| $CH_3$ | $CH_3$ | H | $-COOCH_3$ |
| $CH_3$ | $C_2H_5$ | H | $-COOCH_3$ |
| $C_2H_5$ | $C_2H_5$ | H | $-COOCH_3$ |
| $CH_3$ | $CH_3$ | $3-CH_3$ | $-COOCH_3$ |
| Cl | $CH_3$ | H | $-COOCH_3$ |
| $CH_3$ | $CH_3$ | H | $-CH_2-$ 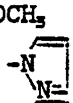 |
| $CH_3$ | $C_2H_5$ | H | $-CH_2-$ 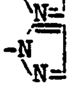 |
| $C_2H_5$ | $C_2H_5$ | H | $-CH_2-$ 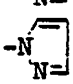 |
| $CH_3$ | $CH_3$ | $3-CH_3$ | $-CH_2-$ 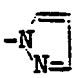 |

| $R^1$ | $R^2$ | $R^3$ | $R^7$ |
|---|---|---|---|
| Cl | $CH_3$ | H | $-CH_2-$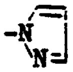 |
| $CH_3$ | $CH_3$ | H | $-CH_2-$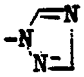 |
| $CH_3$ | $C_2H_5$ | H | $-CH_2-$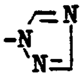 |
| $C_2H_5$ | $C_2H_5$ | H | $-CH_2-$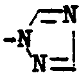 |
| $CH_3$ | $CH_3$ | 3-$CH_3$ | $-CH_2-$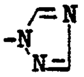 |
| Cl | $CH_3$ | H | $-CH_2-$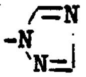 |
| $CH_3$ | $CH_3$ | H | $-CH_2-$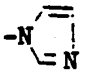 |
| $CH_3$ | $C_2H_5$ | H | $-CH_2-$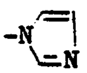 |
| $C_2H_5$ | $C_2H_5$ | H | $-CH_2-$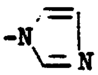 |
| $CH_3$ | $CH_3$ | 3-$CH_3$ | $-CH_2-$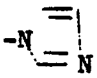 |
| Cl | $CH_3$ | H | $-CH_2-$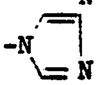 |
| $CH_3$ | $CH_3$ | H | $-CH_2-O-SO_2CH_3$ |
| $CH_3$ | $C_2H_5$ | H | $-CH_2-O-SO_2CH_3$ |
| $C_2H_5$ | $C_2H_5$ | H | $-CH_2-O-SO_2CH_3$ |
| $CH_3$ | $CH_3$ | 3-$CH_3$ | $-CH_2-O-SO_2CH_3$ |
| Cl | $CH_3$ | H | $-CH_2-O-SO_2CH_3$ |
| $CH_3$ | $CH_3$ | H | $-CHCl_2$ |
| $CH_3$ | $C_2H_5$ | H | $-CHCl_2$ |
| $C_2H_5$ | $C_2H_5$ | H | $-CHCl_2$ |
| $CH_3$ | $CH_3$ | 3-$CH_3$ | $-CHCl_2$ |
| Cl | $CH_3$ | H | $-CHCl_2$ |
| $CH_3$ | $CH_3$ | H | 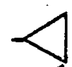 |
| $CH_3$ | $C_2H_5$ | H | 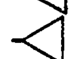 |
| $C_2H_5$ | $C_2H_5$ | H | 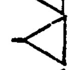 |
| $CH_3$ | $CH_3$ | 3-$CH_3$ | 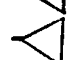 |
| Cl | $CH_3$ | H | 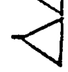 |
| $CH_3$ | $CH_3$ | H | $-OC_2H_5$ |
| $CH_3$ | $C_2H_5$ | H | $-OC_2H_5$ |
| $C_2H_5$ | $C_2H_5$ | H | $-OC_2H_5$ |
| $CH_3$ | $CH_3$ | 3-$CH_3$ | $-OC_2H_5$ |
| Cl | $CH_3$ | H | $-OC_2H_5$ |

| $R^1$ | $R^2$ | $R^3$ | $R^7$ —— |
|---|---|---|---|
| $CH_3$ | $CH_3$ | H | - 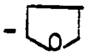 |
| $CH_3$ | $C_2H_5$ | H | - 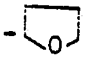 |
| $C_2H_5$ | $C_2H_5$ | H | - 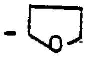 |
| $CH_3$ | $CH_3$ | 3-$CH_3$ | - 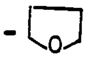 |
| Cl | $CH_3$ | H | - 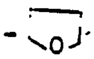 |
| $CH_3$ | $CH_3$ | H | $-CH_2-O-CH_2-O-C_2H_5$ |
| $CH_3$ | $C_2H_5$ | H | $-CH_2-O-CH_2-O-C_2H_5$ |
| $C_2H_5$ | $C_2H_5$ | H | $-CH_2-O-CH_2-O-C_2H_5$ |
| $CH_3$ | $CH_3$ | 3-$CH_3$ | $-CH_2-O-CH_2-O-C_2H_5$ |
| Cl | $CH_3$ | H | $-CH_2-O-CH_2-O-C_2H_5$ |
| $CH_3$ | $CH_3$ | H | $-CH_2-O-$ 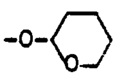 |
| $CH_3$ | $C_2H_5$ | H | $-CH_2-O-$ 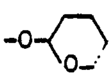 |
| $C_2H_5$ | $C_2H_5$ | H | $-CH_2-O-$ 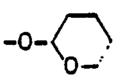 |
| $CH_3$ | $CH_3$ | 3-$CH_3$ | $-CH_2-O-$ 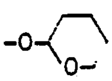 |
| Cl | $CH_3$ | H | $-CH_2-O-$ 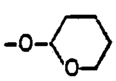 |

Die außerdem für das erfindungsgemäße Verfahren (b) als Ausgangsstoffe zu verwendenden substituierten Oxim-ether sind durch die Formel (V) allgemein definiert. In dieser Formel stehen $R^4$, $R^5$ und $R^6$ vorzugsweise für diejenigen Reste, die bereits im Zusammenhang mit der Beschreibung der erfindungsgemäßen Stoffe der Formel (I) vorzugsweise genannt wurden. Y steht vorzugsweise für Chlor, Brom, den Mesylat- und Tosylat-Rest.

Die substituierten Oxim-ether der Formel (V) sind bekannt (vgl. z. B. US-Patentschrift 3 896 189); bzw. können sie in allgemein bekannter Art und Weise erhalten werden, indem man entsprechende Carbonylverbindungen mit Hydroxylamin-(Derivaten) in Gegenwart eines Lösungsmittels, vorzugsweise eines Alkohols, bei Temperaturen zwischen 20 °C und 100 °C, vorzugsweise zwischen 50 °C und 80 °C umsetzt. Dabei wird das Hydroxylamin-(Derivat) vorzugsweise in Form eines Salzes, insbesondere als Hydrochlorid, gegebenenfalls in Gegenwart eines Säurebindemittels, wie beispielsweise Natriumacetat, eingesetzt. Die Isolierung der Endprodukte erfolgt in üblicher Weise. Die substituierten Oxim-ether der Formel (V) können auch erhalten werden, wenn Oxim-ether der Formel

$$R^4-CH_2-\overset{\displaystyle R^5}{\overset{|}{C}}=N-O-R^6 \qquad \text{(Va)}$$

in welcher $R^4$, $R^5$ und $R^6$ die oben angegebene Bedeutung haben, in üblicher Weise halogeniert (vergleiche hierzu J. Org. Chem. *36* (1971) 3467).

Als Beispiele für die Ausgangsstoffe der Formel (V) seien genannt:

$$(Br)Cl-\overset{\displaystyle R^4}{\overset{|}{C}H}-\overset{\displaystyle R^5}{\overset{|}{C}}=N-O-R^6 \qquad \text{(V)}$$

| $R^4$ | $R^5$ | $R^6$ | $R^4$ | $R^5$ | $R^6$ |
|---|---|---|---|---|---|
| H | H | H | H | $CH_3$ | H |
| H | H | $CH_3$ | H | $CH_3$ | $CH_3$ |
| H | H | $C_2H_5$ | H | $CH_3$ | $C_2H_5$ |
| H | H | n-$C_3H_7$ | H | $CH_3$ | n-$C_3H_7$ |
| H | H | i-$C_3H_7$ | H | $CH_3$ | i-$C_3H_7$ |
| H | H | n-$C_4H_9$ | H | $CH_3$ | n-$C_4H_9$ |
| H | H | tert.-$C_4H_9$ | H | $CH_3$ | tert.-$C_4H_9$ |
| H | H | $-CH_2-CH=CH_2$ | H | $CH_3$ | $-CH_2-CH=CH_2$ |
| H | H | $-CH_2-C\equiv CH$ | H | $CH_3$ | $-CH_2-C\equiv CH$ |
| H | H | $-CH_2-O-CH_3$ | H | $CH_3$ | $-CH_2-O-CH_3$ |
| H | H | $-CH_2-S-CH_3$ | H | $CH_3$ | $-CH_2-S-CH_3$ |
| H | H | $-CH_2$ 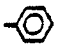 | H | $CH_3$ | $-CH_2$ 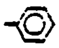 |
| H | H | $-CH_2$ 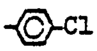 -Cl | H | $CH_3$ | $-CH_2$ 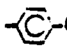 -Cl |
| H | $C_2H_5$ | H | $CH_3$ | H | H |
| H | $C_2H_5$ | $CH_3$ | $CH_3$ | H | $CH_3$ |
| H | $C_2H_5$ | $C_2H_5$ | $CH_3$ | H. | $C_2H_5$ |
| H | $C_2H_5$ | n-$C_3H_7$ | $CH_3$ | H | n-$C_3H_7$ |
| H | $C_2H_5$ | i-$C_3H_7$ | $CH_3$ | H | i-$C_3H_7$ |
| H | $C_2H_5$ | n-$C_4H_9$ | $CH_3$ | H | n-$C_4H_9$ |
| H | $C_2H_5$ | tert.-$C_4H_9$ | $CH_3$ | H | tert.-$C_4H_9$ |
| H | $C_2H_5$ | $-CH_2-CH=CH_2$ | $CH_3$ | H | $-CH_2-CH=CH_2$ |
| H | $C_2H_5$ | $-CH_2-C\equiv CH$ | $CH_3$ | H | $-CH_2-C\equiv CH$ |
| H | $C_2H_5$ | $-CH_2-O-CH_3$ | $CH_3$ | H | $-CH_2-O-CH_3$ |
| H | $C_2H_5$ | $-CH_2-S-CH_3$ | $CH_3$ | H | $-CH_2-S-CH_3$ |
| H | $C_2H_5$ | $-CH_2$ 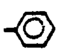 | $CH_3$ | H | $-CH_2$ 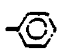 |
| H | $C_2H_5$ | $-CH_2$ 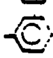 -Cl | $CH_3$ | H | $-CH_2$ 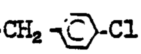 -Cl |
| $CH_3$ | $CH_3$ | H | $C_2H_5$ | H | H |
| $CH_3$ | $CH_3$ | $CH_3$ | $C_2H_5$ | H | $CH_3$ |
| $CH_3$ | $CH_3$ | $C_2H_5$ | $C_2H_5$ | H | $C_2H_5$ |
| $CH_3$ | $CH_3$ | n-$C_3H_7$ | $C_2H_5$ | H | n-$C_3H_7$ |
| $CH_3$ | $CH_3$ | i-$C_3H_7$ | $C_2H_5$ | H | i-$C_3H_7$ |
| $CH_3$ | $CH_3$ | n-$C_4H_9$ | $C_2H_5$ | H | n-$C_4H_9$ |
| $CH_3$ | $CH_3$ | tert.-$C_4H_9$ | $C_2H_5$ | H | tert.-$C_4H_9$ |
| $CH_3$ | $CH_3$ | $-CH_2-CH=CH_2$ | $C_2H_5$ | H | $-CH_2-CH=CH_2$ |
| $CH_3$ | $CH_3$ | $-CH_2-C\equiv CH$ | $C_2H_5$ | H | $-CH_2-C\equiv CH$ |
| $CH_3$ | $CH_3$ | $-CH_2-O-CH_3$ | $C_2H_5$ | H | $-CH_2-O-CH_3$ |
| $CH_3$ | $CH_3$ | $-CH_2-S-CH_3$ | $C_2H_5$ | H | $-CH_2-S-CH_3$ |
| $CH_3$ | $CH_3$ | $-CH_2$ 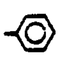 | $C_2H_5$ | H | $-CH_2$ 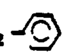 |
| $CH_3$ | $CH_3$ | $-CH_2$ 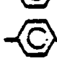 -Cl | $C_2H_5$ | H | $-CH_2$ 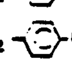 -Cl |
| $C_2H_5$ | $CH_3$ | H | | | |
| $C_2H_5$ | $CH_3$ | $CH_3$ | | | |
| $C_2H_5$ | $CH_3$ | $C_2H_5$ | | | |
| $C_2H_5$ | $CH_3$ | n-$C_3H_7$ | | | |
| $C_2H_5$ | $CH_3$ | i-$C_3H_7$ | | | |
| $C_2H_5$ | $CH_3$ | n-$C_4H_9$ | | | |

| $R^4$ | $R^5$ | $R^6$ | $R^4$ | $R^5$ | $R^6$ |
|---|---|---|---|---|---|
| $C_2H_5$ | $CH_3$ | tert.-$C_4H_9$ | | | |
| $C_2H_5$ | $CH_3$ | $-CH_2-CH=CH_2$ | | | |
| $C_2H_5$ | $CH_3$ | $-CH_2-C\equiv CH$ | | | |
| $C_2H_5$ | $CH_3$ | $-CH_2-O-CH_3$ | | | |
| $C_2H_5$ | $CH_3$ | $-CH_2-S-CH_3$ | | | |
| $C_2H_5$ | $CH_3$ | $-CH_2-$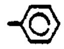 | | | |
| $C_2H_5$ | $CH_3$ | $-CH_2-$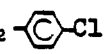$-Cl$ | | | |

Die bei der Durchführung des erfindungsgemäßen Verfahrens (c) als Ausgangsstoffe benötigten N-substituierten Anilide sind durch die Formel (VI) allgemein definiert. In dieser Formel stehen $R^1$, $R^2$, $R^3$, $R^4$, $R^5$ und $R^7$ vorzugsweise für diejenigen Reste, die bereits im Zusammenhang mit der Beschreibung der erfindungsgemäßen Stoffe der Formel (I) vorzugsweise für diese Substituenten genannt wurden.

Die N-substituierten Anilide der Formel (VI) sind noch nicht bekannt. Sie lassen sich jedoch in einfacher Weise nach mehreren Verfahren herstellen, indem man z. B.:

α) Anilide der Formel

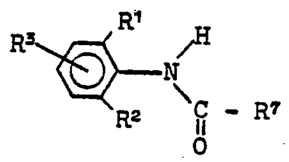

(IV)

in welcher $R^1$, $R^2$, $R^3$ und $R^7$ die oben angegebene Bedeutung haben,
mit Keto-Derivaten der Formel

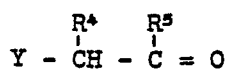

(XVI)

in welcher $R^4$, $R^5$ und Y die oben angegebene Bedeutung haben,
in Gegenwart eines Säurebinders und in Gegenwart eines Verdünnungsmittels umsetzt, oder

β) Anilide der Formel

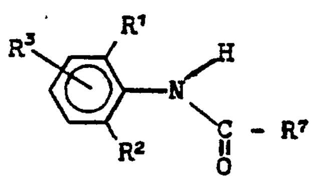

(IV)

in welcher $R^1$, $R^2$, $R^3$ und $R^7$ die oben angegebene Bedeutung haben,
mit Propargylhalogeniden der Formel

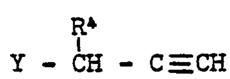

(XVII)

in welcher $R^4$ und Y die oben angegebene Bedeutung haben,
in Gegenwart eines Säurebinders und gegebenenfalls in Gegenwart eines Verdünnungsmittels umsetzt und die entstehenden Propargyl-acetanilide der Formel

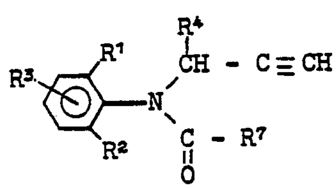

(XVIII)

in welcher $R^1$, $R^2$, $R^3$, $R^4$ und $R^7$ die oben angegebene Bedeutung haben,
in üblicher Weise hydratisiert, oder

γ) Anilin-Derivate der Formel

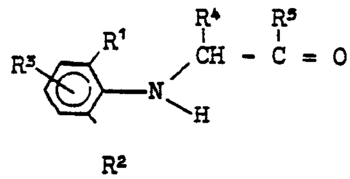

(XIX)

in welcher $R^1$, $R^2$, $R^3$, $R^4$ und $R^5$ die oben angegebene Bedeutung haben, mit bekannten Säurechloriden oder -bromiden der Formel

$$R^7 - \underset{\underset{O}{\|}}{C} - Cl/Br) \qquad (IIIa)$$

in welcher $R^7$ die oben angegebene Bedeutung hat, in Gegenwart eines Verdünnungsmittels und gegebenenfalls in Gegenwart eines Säurebindemittels umsetzt.

Bei der Herstellung der N-substituierten Acetanilide der Formel (VI) nach den Verfahren (α), (β) und (γ) können als Säurebindemittel alle üblichen Säureakzeptoren verwendet werden. Vorzugsweise in Betracht kommen Alkalicarbonate, wie Kalium- oder Natriumcarbonat.

Als Verdünnungsmittel können bei den Verfahren (α), (β) und (γ) alle üblichen inerten organischen Lösungsmittel eingesetzt werden. Vorzugsweise in Betracht kommen aromatische Lösungsmittel, wie insbesondere Toluol, sowie Dimethylformamid.

Nach einer bevorzugten Ausführungsform wird die Umsetzung gemäß Verfahren (α) und (β) in einem Zweiphasensystem, wie beispielsweise wässrige Natron- oder Kalilauge/Toluol oder Methylenchlorid, gegebenenfalls unter Zusatz von 0,1-1 Mol eines Phasentransferkatalysators, wie beispielsweise einer Ammonium- oder Phosphoniumverbindung, durchgeführt.

Die Reaktionstemperaturen können bei den Verfahren (α), (β) und (γ) innerhalb eines größeren Bereiches variiert werden. Im allgemeinen arbeitet man zwischen 0 °C und 180 °C, vorzugsweise zwischen 20 °C und 160 °C.

Bei der Durchführung gemäß Verfahren (α), (β) und (γ) arbeitet man vorzugsweise in äquimolaren Mengen. Die Aufarbeitung und Isolierung der Reaktionsprodukte erfolgt nach üblichen Methoden.

In manchen Fällen erweist es sich als vorteilhaft, die N-substituierten Acetanilide der Formel (VI) durch saure Hydrolyse der erfindungsgemäßen Verbindungen der Formel (I) herzustellen.

Die außerdem für das erfindungsgemäße Verfahren (c) als Ausgangsstoffe zu verwendenden Hydroxylamin-(Derivate) sind durch die Formel (VIII) allgemein definiert. In dieser Formel steht $R^6$ vorzugsweise für diejenigen Reste, die bereits im Zusammenhang mit der Beschreibung der erfindungsgemäßen Stoffe der Formel (I) vorzugsweise für diesen Substituenten genannt wurden. Die Verbindungen der Formel (VII) werden vorzugsweise in Form ihrer Hydrohalogenide, wie insbesondere als Hydrochlorid, eingesetzt.

Die bei der Durchführung des erfindungsgemäßen Verfahrens (d) neben den erfindungsgemäßen Oximen der Formel (VIII) als Ausgangsstoffe benötigten Verbindungen sind durch die Formel (IX) allgemein definiert. In dieser Formel steht $R^9$ vorzugsweise für geradkettiges oder verzweigtes Alkyl mit 1 bis 4 Kohlenstoffatomen, Alkenyl und Alkinyl mit jeweils 2 bis 4 Kohlenstoffatomen, Alkoxyalkyl und Alkylthioalkyl mit jeweils 1 bis 4 Kohlenstoffatomen im Alkylteil und 1 bis 4 Kohlenstoffatomen im Alkoxy- bzw. im Alkylthio-Teil, sowie für gegebenenfalls substituiertes Aralkyl mit 6 bis 10 Kohlenstoffatomen im Arylteil und 1 bis 2 Kohlenstoffatomen, im Alkylteil, wobei vorzugsweise Benzyl genannt sei, und als Substituenten vorzugsweise die bei $R^6$ bereits vorzugsweise genannten Arylsubstituenten in Frage kommen.

Die Hydroxylamin-(Derivate) der Formel (VII) und die Verbindungen der Formel (IX) sind allgemein bekannte Verbindungen der organischen Chemie.

Die bei der Durchführung des erfindungsgemäßen Verfahrens (e) als Ausgangsstoffe benötigten Halogen-acetanilide sind durch die Formel (X) allgemein definiert. In dieser Formel stehen $R^1$, $R^2$, $R^3$, $R^4$, $R^5$ und $R^6$ vorzugsweise für diejenigen Reste, die bereits im Zusammenhang mit der Beschreibung der erfindungsgemäßen Stoffe der Formel (I) vorzugsweise für diese Substituenten genannt wurden.

Die Halogenacetanilide der Formel (X) sind noch nicht bekannt. Sie sind jedoch Gegenstand einer eigenen, älteren Anmeldung, die noch nicht bekannt ist (vergleiche die Deutsche Patentanmeldung 28 47 827 [Le A 19 240] vom 3.11.1978). Sie können nach den dort angegebenen Verfahren erhalten werden, indem man z. B. Anilinoalkyloxim-ether der Formel (II) mit Halogen-essigsäurechloriden oder -bromiden bzw. -anhydriden gemäß Verfahren (a) umsetzt; oder indem man Halogen-acetanilide mit substituierten Oxim-ethern der Formel (V) gemäß Verfahren (b) umsetzt.

Die außerdem für das erfindungsgemäße Verfahren (e) noch als Ausgangsstoffe zu verwendenden Verbindungen sind durch die Formel (XI) allgemein definiert. In dieser Formel stehen Az und $R^8$ vorzugsweise für diejenigen Reste, die bereits im Zusammenhang mit der Beschreibung der erfindungsgemäßen Stoffe der Formel (I) vorzugsweise genannt wurden. B steht vorzugsweise für Wasserstoff, Natrium und Kalium.

Die Verbindungen der Formel (XI) sind allgemein bekannte Verbindungen der organischen Chemie.

Die bei der Durchführung des erfindungsgemäßen Verfahrens (f) als Ausgangsstoffe benötigten Hydroxyacetanilide sind durch die Formel (XII) allgemein definiert. In dieser Formel stehen, $R^1$, $R^2$, $R^3$, $R^4$, $R^5$ und $R^6$ vorzugsweise für diejenigen Reste, die bereits im Zusammenhang mit der Beschreibung der erfindungsgemäßen Stoffe der Formel (I) vorzugsweise für diese Substituenten genannt wurden.

Die Hydroxyacetanilide der Formel (XII) können auf allgemein bekannte Art und Weise erhalten werden, indem man Acyloxyacetylanilide der Formel

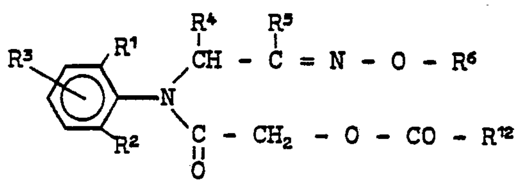

(XVII)

in welcher

$R^1$, $R^2$, $R^3$, $R^4$, $R^5$ und $R^6$ die oben angegebene Bedeutung haben und

$R^{12}$ für Alkyl mit 1 bis 4 Kohlenstoffatomen steht,

mit Natron- oder Kalilauge bzw. mit einem Alkalialkoholat eines niederen Alkohols wie z. B. Natriummethylat oder Natriumethylat, bei Temperaturen zwischen 20 und 40 °C verseift und nach Ansäuern in üblicher Weise die Verbindungen der Formel (XII) isoliert.

Die Acyloxyacetylanilide der Formel (XVII) können in allgemein üblicher und bekannter Art und Weise erhalten werden, indem man z. B. bei Halogenacetaniliden der Formel (X) den reaktionsfähigen Substituenten Hal durch Reaktion mit einer niederen Alkancarbonsäure austauscht, wobei die Säure vorzugsweise in Form ihrer Alkali- oder Erdalkalisalze eingesetzt wird, oder indem man Anilinoalkyl-oximether der Formel (II) mit entsprechenden Acyl-oxyacetylhalogeniden umsetzt.

Die außerdem für das erfindungsgemäße Verfahren (f/1) als Ausgangsstoffe zu verwendenden Halogenide sind durch die Formel (XIII) allgemein definiert. In dieser Formel steht $R^8$ vorzugsweise für die Reste, die bereits im Zusammenhang mit der Beschreibung der erfindungsgemäßen Stoffe der Formel (I) vorzugsweise für diesen Substituenten genannt wurden. Hal steht vorzugsweise für Chlor, Brom oder Jod.

Die Halogenide der Formel (XIII) sind allgemein bekannte Verbindungen der organischen Chemie.

Das für das erfindungsgemäße Verfahren (f/2) außerdem als Ausgangsstoffe zu verwendende Dihydropyran ist ebenfalls eine bekannte Verbindung der organischen Chemie.

Als Verdünnungsmittel kommen für die erfindungsgemäße Umsetzung gemäß Verfahren (a) vorzugsweise inerte organische Lösungsmittel in Frage. Hierzu gehören vorzugsweise Ketone, wie Diethylketon, insbesondere Aceton und Methylethylketon ; Nitrile, wie Propionitril, insbesondere Acetonitril ; Ether, wie Tetrahydrofuran oder Dioxan ; aliphatische und aromatische Kohlenwasserstoffe, wie Petrolether, Benzol, Toluol oder Xylol ; halogenierte Kohlenwasserstoffe, wie Methylenchlorid, Tetrachlorkohlenstoff, Chloroform oder Chlorbenzol ; und Ester, wie Essigester.

Das erfindungsgemäße Verfahren (a) kann gegebenenfalls in Gegenwart von Säurebindern (Halogenwasserstoff-Akzeptoren) durchgeführt werden. Als solche können alle üblichen Säurebindemittel verwendet werden. Hierzu gehören vorzugsweise organische Basen, wie tertiäre Amine, beispielsweise Triethylamin, oder Pyridin, ferner anorganische Basen, wie beispielsweise Alkalihydroxide und Alkalicarbonate.

Die Reaktionstemperaturen können bei der Durchführung des erfindungsgemäßen Verfahrens (a) in einem größeren Bereich variiert werden. Im allgemeinen arbeitet man zwischen 0 °C und 120 °C, vorzugsweise zwischen 20 und 100 °C.

Bei der Durchführung des erfindungsgemäßen Verfahrens (a) setzt man vorzugsweise auf 1 Mol der Verbindungen der Formel (II) 1 bis 1,5 Mol Acylierungsmittel und 1 bis 1,5 Mol Säurebinder ein. Die Isolierung der Verbindungen der Formel (I) erfolgt in üblicher Weise.

Als Verdünnungsmittel kommen für die erfindungsgemäße Umsetzung gemäß Verfahren (b) vorzugsweise inerte organische Lösungsmittel infrage. Hierzu gehören vorzugsweise die bei dem Verfahren (a) bereits genannten Solventien.

Als Säurebindemittel kommen bei dem Verfahren (b) vorzugsweise die bei dem Verfahren (a) bereits genannten Stoffe in Frage.

Die Reaktionstemperaturen können bei der Durchführung des erfindungsgemäßen Verfahrens (b) in einem größeren Bereich variiert werden. Im allgemeinen arbeitet man zwischen 0 °C und 150 °C, vorzugsweise zwischen 20 °C und 100 °C.

Bei der Durchführung des erfindungsgemäßen Verfahrens (b) arbeitet man vorzugsweise mit äquimolaren Mengen. Es ist jedoch auch möglich, eine der Komponenten in einem Ueberschuß einzusetzen. Die Aufarbeitung und Isolierung der Reaktionsprodukte erfolgt nach üblichen Methoden.

In einer bevorzugten Ausführungsform wird die erfindungsgemäße Umsetzung gemäß Verfahren (b) in einem Zweiphasensystem, wie beispielsweise wässrige Natron- oder Kalilauge/Toluol oder Methylenchlorid, gegebenenfalls unter Susatz von 0,1-1 Mol eines Phasen-transfer-Katalysators, wie beispielsweise einer Ammonium- oder Phosphoniumverbindung, beispielsweise seien Benzyl-dodecyl-dimethylammoniumchlorid und Triethyl-benzyl-ammoniumchlorid genannt, durchgeführt.

Als Verdünnungsmittel kommen für die erfindungsgemäße Umsetzung gemäß Verfahren (c) vorzugsweise Alkohole bzw. wäßrige Alkohole in Frage.

Werden die Verbindungen der Formel (VII) bei der Durchführung des Verfahrens (c) in Form ihrer Salze, vorzugsweise als Hydrochloride, eingesetzt, so wird in Gegenwart eines Säurebindemittels gearbeitet. Hierzu gehören vorzugsweise Alkalicarbonate und -acetate.

Die Reaktionstemperaturen können bei der Durchführung des erfindungsgemäßen Verfahrens (c) in einem größeren Bereich variiert werden. Im allgemeinen arbeitet man zwischen 20 und 120 °C, vorzugsweise zwischen 40 °C und 100 °C.

Bei der Durchführung des erfindungsgemäßen Verfahrens (c) arbeitet man vorzugsweise mit äquimolaren Mengen. Es ist jedoch auch möglich, eine der Komponenten in einem Ueberschuß einzusetzen. Die Aufarbeitung und Isolierung der Reaktionsprodukte erfolgt nach üblichen Methoden.

Als Verdünnungsmittel kommen für die erfindungsgemäße Umsetzung gemäß Verfahren (d) alle inerten organischen Lösungsmittel infrage. Hierzu gehören vorzugsweise Ether, wie Diethylether und Dioxan ; aromatische Kohlenwasserstoffe, wie Toluol und Benzol ; in einzelnen Fällen auch chlorierte Kohlenwasserstoffe, wie Chloroform, Methylenchlorid und Tetrachlorkohlenstoff ; sowie Hexamethylphosphorsäure-triamid und Dimethylformamid.

Die Reaktionstemperaturen können bei der Durchführung des erfindungsgemäßen Verfahrens (d) in einem größeren Bereich variiert werden. Im allgemeinen arbeitet man zwischen 20 und 150 °C, vorzugsweise bei Raumtemperatur. In einzelnen Fällen ist es vorteilhaft, bei der Siedetemperatur des Lösungsmittels, beispielsweise zwischen 60 und 100 °C, zu arbeiten.

Bei der Durchführung des erfindungsgemäßen Verfahrens (d) setzt man auf 1 Mol Alkalisalz eines Oxims der Formel (VIII) vorzugsweise 1 bis 3 Mol an Verbindung der Formel (IX) ein. Die Aufarbeitung und Isolierung der Reaktionsprodukte erfolgt nach üblichen Methoden.

In einer bevorzugten Ausführungsform des Verfahrens (d) wird zweckmäßigerweise so verfahren, daß man von einem Oxim der Formel (VIII) ausgeht, letzteres in einem geeigneten inerten organischen Lösungsmittel mittels Alkalimetall-hydrid oder -amid in das Salz überführt, und letzteres ohne Isolierung sofort mit einem Halogenid der Formel (IX) umsetzt, wobei unter Austritt von Alkalihalogenid die erfindungsgemäßen Verbindungen der Formel (I) in einem Arbeitsgang erhalten werden.

Nach einer weiteren bevorzugten Ausführungsform des Verfahrens (d) werden zweckmäßigerweise die Herstellung der Salze der Oxime der Formel (VIII) sowie die erfindungsgemäße Umsetzung in einem Zweiphasensystem, wie beispielsweise wässrige Natron- oder Kalilauge/Toluol oder Methylenchlorid, unter Zusatz von 0,1-1 Mol eines Phasen-transfer-katalysators, wie beispielsweise einer Ammonium- oder Phosphoniumverbindung, durchgeführt.

Als Verdünnungsmittel kommen für die erfindungsgemäße Umsetzung gemäß Verfahren (e) vorzugsweise inerte organische Lösungsmittel infrage. Hierzu gehören vorzugsweise die beim Verfahren (a) bereits genannten Lösungsmittel.

Die Umsetzung nach Verfahren (e) kann gegebenenfalls in Gegenwart eines Säurebinders durchgeführt werden. Man kann alle üblicherweise verwendbaren anorganischen oder organischen Säurebinder zugeben, wie Alkalicarbonate, beispielsweise Natriumcarbonat, Kaliumcarbonat und Natriumhydrogencarbonat, oder wie niedere tertiäre Alkylamine, Cycloalkylamine oder Aralkylamine, beispielsweise Triethylamin, Dimethylbenzylamin ; oder wie Pyridin und Diazabicyclooctan. Vorzugsweise verwendet man einen Ueberschuß an Azol.

Die Reaktionstemperaturen können beim Verfahren (e) in einem größeren Bereich variiert werden. Im allgemeinen arbeitet man zwischen etwa 20 bis 150 °C, vorzugsweise bei 60 bis 120 °C. Bei Anwesenheit eines Lösungsmittels wird zweckmäßigerweise beim Siedepunkt des jeweiligen Lösungsmittels gearbeitet.

Bei der Durchführung des erfindungsgemäßen Verfahrens (e) setzt man auf 1 Mol der Verbindungen der Formel (X) vorzugsweise 1 bis 2 Mol der Verbindungen der Formel (XI) und gegebenenfalls 1 bis 2 Mol Säurebinder ein. Die Isolierung der Verbindungen der Formel (I) erfolgt in üblicher Weise.

Als Verdünnungsmittel kommen für die erfindungsgemäße Umsetzung gemäß Verfahren (f) vorzugsweise inerte organische Lösungsmittel infrage. Hierzu gehören vorzugsweise die beim Verfahren (a) bereits genannten Lösungsmittel.

Die Umsetzung nach Verfahren (f/1) kann gegebenenfalls in Gegenwart eines Säurebinders durchgeführt werden. Man kann alle üblicherweise verwendbaren anorganischen oder organischen Säurebinder zugeben. Hierzu gehören vorzugsweise die beim Verfahren (a) bereits genannten Verbindungen.

Die Reaktionstemperaturen können beim Verfahren (f/1) in einem größeren Bereich variiert werden. Im allgemeinen arbeitet man zwischen 20 und 150 °C, vorzugsweise bei der Siedetemperatur des Lösungsmittels, beispielsweise zwischen 60 und 100 °C.

Bei der Durchführung des erfindungsgemäßen Verfahrens (f/1) setzt man auf 1 Mol der Verbindungen der Formel (XII), gegebenenfalls nach Zugabe von 1 bis 2 Mol einer starken Base, wie z. B. eines Alkalihydrids, 1 Mol Halogenid der Formel (XIII) und gegebenenfalls 1 bis 2 Mol Säurebinder ein. Zur Isolierung der Endprodukte wird das Reaktionsgemisch vom Lösungsmittel befreit und der

Rückstand mit Wasser und einem organischen Lösungsmittel versetzt. Die organische Phase wird abgetrennt und in üblicher Weise aufgearbeitet.

Nach einer bevorzugten Ausführungsform des Verfahrens (f/1) wird zweckmäßigerweise so verfahren, daß man von einem Hydroxyacetanilid der Formel (XII) ausgeht, letzteres in einem geeigneten inerten Lösungsmittel mittels Alkalimetall-hydrid oder -bromid in das Alkali-metall-alkanolat überführt, und letzteres ohne Isolierung sofort mit einem Halogenid der Formel (XIII) umsetzt, wobei unter Austritt von Alkalihalogenid die erfindungsgemäßen Verbindungen der Formel (I) in einem Arbeitsgang erhalten werden.

Die Umsetzung nach Verfahren (f/2) kann gegebenenfalls in Gegenwart eines Katalysators durchgeführt werden. Hierzu verwendet man vorzugsweise Chlorwasserstoff (vgl. J. Am. Chem. Soc. *69,* 2246 (1947), ibid. *70,* 4187 (1948)).

Die Reaktionstemperaturen können beim Verfahren (f/2) in einem größeren Bereich variiert werden. Im allgemeinen arbeitet man zwischen 0 und 100 °C, vorzugsweise zwischen 20 und 60 °C.

Bei der Durchführung des erfindungsgemäßen Verfahrens (f/2) arbeitet man vorzugsweise in molaren Mengen, Die Isolierung der Verbindungen der Formel (I) erfolgt in üblicher Weise.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Oomyceten, z. B. gegen den Erreger der Kraut- und Braunfäule der Tomate und Kartoffel (Phytophthora infestans) sowie von Pyricularia oryzae am Reis, eingesetzt werden. Besonders hervorzuheben ist, daß die erfindungsgemäßen Wirkstoffe nicht nur eine protektive, sondern auch eine kurativ/eradikative Wirkung entfalten. Außerdem besitzen sie systemische Eigenschaften. So gelingt es, Pflanzen gegen Pilzbefall zu schützen, wenn man den Wirkstoff über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid ; als feste Trägerstoffe kommen in Frage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate ; als Dispergiermittel kommen in Frage : z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff,

vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Volgelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,000 1 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,000 01 bis 0,1 Gew.-%, vorzugsweise von 0,000 1 bis 0,02 %, am Wirkungsort erforderlich.

In den nachfolgenden Beispielen wird die nachstehend angegebene Verbindung als Vergleichssubstanz eingesetzt :

(A) 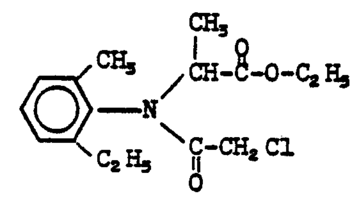

N-Chloroacetyl-N-(2-ethyl-6-methylphenyl)-alaninethylester

Beispiel A

Phytophthora-Test (Tomaten)/Protektiv

Lösungsmittel : 4,7 Gewichtsteile Aceton
Emulgator : 0,3 Gewichtsteile Alkyl-aryl-polyglykol-ether
Wasser : 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Tomatenpflanzen mit 2 bis 4 Laubblättern bis zur Tropfnässe. Die Pflanzen verbleiben 24 Stunden bei 20 °C und einer relativen Luftfeuchtigkeit von 70 % im Gewächshaus. Anschließend werden die Tomatenpflanzen mit einer wässrigen Sporensuspension von Phytophthora infestans inokuliert. Die Pflanzen werden in eine Feuchtkammer mit einer 100 %igen Luftfeuchtigkeit und einer Temperatur von 13 bis 20 °C gebracht.

Nach 5 Tagen wird der Befall der Tomatenpflanzen bestimmt. Die erhaltenen Boniturwerte werden auf Prozent Befall umgerechnet. 0 % bedeutet keinen Befall, 100 % bedeutet, daß die Pflanzen vollständig befallen sind.

In diesem Test zeigen z. B. folgende Verbindungen eine sehr gute Wirkung, die derjenigen der aus dem Stand der Technik bekannten Verbindung (A) deutlich überlegen ist :

Verbindungen gemäß Herstellungsbeispielen 5, 3, 6, 8, und 1.

Beispiel B

Phytophthora-Test (Tomaten)/Systemisch

Lösungsmittel : 4,7 Gewichtsteile Aceton
Dispergiermittel : 0,3 Gewichtsteile Alkyl-aryl-polyglykol-ether
Wasser : 95,0 Gewichtsteile

Man vermischt die für die gewünschte Wirkstoffkonzentration in der Gießflüssigkeit nötige Wirkstoffmenge mit der angegebenen Menge des Lösungsmittels und verdünnt das Konzentrat mit der angegebenen Menge Wasser, welches die genannten Zusätze enthält.

In Einheitserde angezogene Tomatenpflanzen mit 2 bis 4 Laubblättern werden mit 10 ml der Gießflüssigkeit in der angegebenen Wirkstoffkonzentration, bezogen auf 100 ml Erde, gegossen.

Die so behandelten Pflanzen werden nach der Behandlung mit einer wässrigen Sporensuspension von Phytophthora infestans inokuliert. Die Pflanzen werden in eine Feuchtkammer mit einer Luftfeuchtigkeit von 100 % und einer Temperatur von 18 bis 20 °C gebracht. Nach 5 Tagen wird der Befall der Tomatenpflanzen bestimmt. Die so erhaltenen Boniturwerte werden auf Prozent Befall umgerechnet. 0 % bedeutet keinen Befall, 100 % bedeutet, daß die Pflanzen vollständig befallen sind.

In diesem Test zeigen z. B. folgende Verbindungen eine sehr gute Wirkung, die derjenigen der aus dem Stand der Technik bekannten Verbindung(A)deutlich überlegen ist :

Verbindungen gemäß Herstellungsbeispielen 3, 2, 8 und 1.

## Herstellungsbeispiele

### Beispiel 1

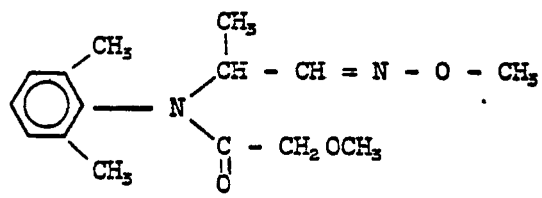

(Verfahren a)

10,3 g (0,05 Mol) N-(1-Methoximino-prop-2-yl)-2,6-dimethyl-anilin und 5 g(0,063 Mol) wasserfreies Pyridin werden in 100 ml wasserfreiem Tetrahydrofuran gelöst und unter Rühren bei 60 °C mit 6,8 g (0.063 Mol) Methoxyessigsäurechlorid versetzt. Es wird noch 1 Stunde unter Rückfluß erhitzt, anschließend das Lösungsmittel im Vakuum abdestilliert und der Rückstand mit Wasser/Methylenchlorid aufgenommen. Die organische Phase wird abgetrennt, über Natriumsulfat getrocknet und eingeengt. Der ölige Rückstand wird im Vakuum in einer Kugelrohrapparatur destilliert. Man erhält 11,5 g (83 % der Theorie) 2,6-Dimethyl-N-(1-methox-imino-prop-2-yl)-N-methoxyacetyl-anilin vom Siedepunkt 140 °C/26,66 Pa und mit einem Brechungsindex von $n_D^{20}$ = 1,526 1.

### Herstellung des Ausgangsproduktes

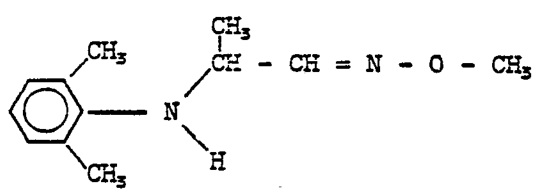

42,4 g (0,35 Mol) 2,6-Dimethylanilin und 25 g (0,17 Mol) gepulvertes Kaliumcarbonat werden in 100 ml Dimethylformamid unter Rühren auf 80 °C erhitzt und tropfenweise mit 29 g (0,17 Mol) 2-Brom-1-methoximino-propan versetzt. Die Temperatur steigt hierbei auf 95 °C an. Es wird noch 2 Stunden bei 80 °C gerührt, das anorganische Salz abgesaugt und das Filtrat destilliert. Nach Abdestillieren des Lösungsmittels und des überschüssigen 2,6-Dimethylanilins erhält man 25,4 g (72,5 % der Theorie) N-(1-Methoximino-prop-2-yl)-2,6-dimethylanilin vom Siedepunkt 107-109 °C/13,33 Pa.

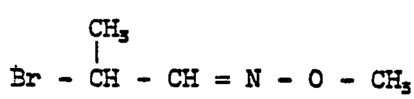

51,9 g (0,6 Mol) 1-Methoximino-propan und 106,7 g (0,6 Mol) N-Brom-succinimid werden in 400 ml Tetrachlorkohlenstoff zum Sieden erhitzt und unter UV-Bestrahlung 2,5 Stunden unter Rückfluß erhitzt. Nach Abkühlen und Abfiltrieren des Succinimids wird das Filtrat zunächst unter Normaldruck und dann am Wasserstrahlvakuum destilliert. Man erhält 68 g (68 % der Theorie) 2-Brom-1-methoximino-propan vom Siedepunkt 49-50 °C/2 666,4 Pa mit einem Brechungsindex von $n_D^{20}$ = 1,475 0.

Beispiel 2

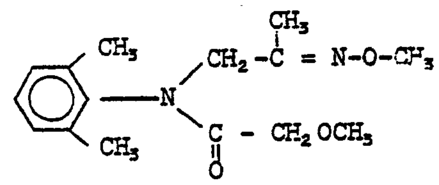

(Verfahren b)

20 g (0,08 Mol) 2,6-Dimethyl-N-methoxyacetyl-N-acetonyl-anilin, 7,5 g (0,09 Mol) O-Methyl-hydroxylamin-hydrochlorid und 9,1 g (0,09 Mol) Triethylamin werden in 100 ml Ethanol 5 Stunden unter Rückfluß erhitzt. Nach Abdestillieren des Lösungsmittels in Vakuum wird der Rückstand zwischen Wasser/Methylenchlorid verteilt. Die organische Phase wird abgetrennt, über Natriumsulfat getrocknet und eingeengt. Der Rückstand kristallisiert nach Zugabe von Petrolether. Man erhält 21 g (94 % der Theorie) 2,6-Dimethyl-N-(2-methoximino-prop-1-yl)-N-methoxyacetyl-anilin vom Schmelzpunkt 56-57 °C.

Herstellung des Ausgangsproduktes

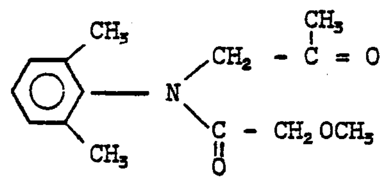

23,1 g (0,1 Mol) 2,6-Dimethyl-N-methoxyacetyl-N-propargyl-anilin werden in 150 ml 85 %iger Ameisensäure auf 80 °C erhitzt und die Reaktionsmischung wird anschließend mit 0,5 g Quecksilbersulfat versetzt. Es wird noch 6 Stunden bei 80 °C gerührt, die Lösung nach Abkühlen mit gesättigter Ammoniumsulfat-Lösung versetzt und mit Methylenchlorid extrahiert. Nach Neutralisation der Methylenchloridphase mit Natriumhydrogencarbonat-Lösung wird die organische Phase mit Natriumsulfat getrocknet und eingeengt. Man erhält 20,5 g (77 % der Theorie) 2,6-Dimethyl-N-acetonyl-N-methoxyacetyl-anilin vom Schmelzpunkt 56-57 °C.

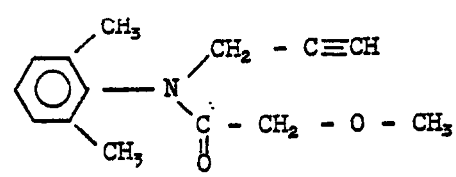

30 g (0,155 Mol) 2,6-Dimethyl-N-methoxyacetyl-anilin und 0,3 g Triethyl-benzyl-ammoniumchlorid werden in einem Zweiphasensystem-Gemisch aus 100 ml 50 %iger Natronlauge und 250 ml Toluol gelöst und unter heftigem Rühren mit 19 g (0,016 Mol) Propargylbromid versetzt. Man läßt 4 Stunden rühren, trennt die Toluolphase ab, wäscht sie mehrfach mit Wasser, trocknet über Natriumsulfat und engt durch Abdestillieren des Lösungsmittels im Wasserstrahlvakuum ein. Der Rückstand wird im Hochvakuum destilliert. Man erhält 26,5 g (74 % der Theorie) 2,6-Dimethyl-N-methoxyacetyl-N-propargyl-anilin vom Siedepunkt 140°/66,66 Pa und vom Schmelzpunkt 49-51 °C.

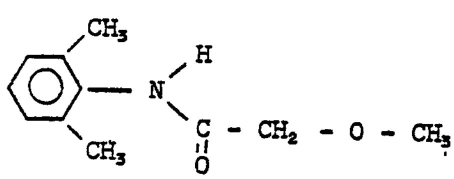

55 g (0,5 Mol) Methoxyacetylchlorid werden bei 5 bis 15 °C unter Rühren und Kühlen in eine Lösung von 61 g (0,5 Mol) 2,6-Dimethylanilin und 50,5 g (0,5 Mol) Triethylamin in 250 ml Toluol getropft. Man läßt 2 Stunden bei 20 °C nachrühren, filtriert, engt ein und nimmt den Rückstand mit Wasser auf. Man extrahiert mit Methylenchlorid, trocknet über Natriumsulfat und engt durch Abdestillieren des Lösungsmittels ein. Der Rückstand wird im Hochvakuum destilliert. Man erhält 55 g (57 % der Theorie) 2,6-Dimethyl-N-methoxyacetyl-anilin vom Siedepunkt 122-132 °/13,33 Pa und vom Schmelzpunkt 61-63 °C.

Beispiel 3

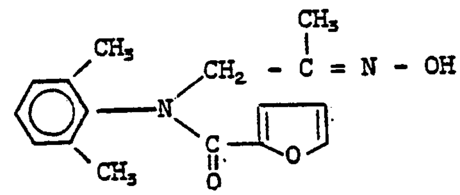

(Verfahren c)

27,1 g (0,1 Mol) 2,6-Dimethyl-N-acetonyl-N-(2-furoyl)-anilin, 14 g (0,2 Mol) Hydroxylammoniumchlorid und 15 ml Triethylamin werden in 150 ml Ethanol 3 Stunden unter Rückfluß erhitzt. Anschließend wird die Reaktionsmischung im Vakuum weitgehend vom Lösungsmittel befreit, der Rückstand mit Methylenchlorid aufgenommen, mit Wasser gewaschen, über Natriumsulfat getrocknet und eingeengt. Der kristalline Rückstand wird mit Ether verrieben und abgesaugt. Man erhält 24,4 g (85 % der Theorie) 2,6-Dimethyl-N-(2-furoyl)-N-(2-hydroximino-prop-1-yl)-anilin vom Schmelzpunkt 155-156 °C.

Herstellung des Ausgangsproduktes

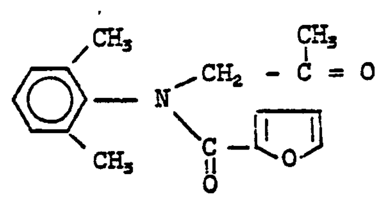

Entsprechend Beispiel 2 durch Umsetzung von 2,6-Dimethyl-N-(2-furoyl)-N-propargyl-anilin. Man erhält in 84 %iger Ausbeute 2,6-Dimethyl-N-acetonyl-N-(2-furoyl)-anilin vom Schmelzpunkt 90-91 °C.

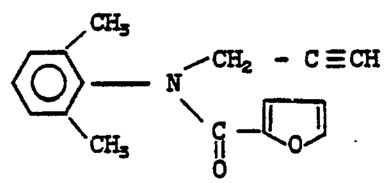

15,9 g (0,1 Mol) 2,6-Dimethyl-N-propargyl-anilin und 8 g (0,1 Mol) Pyridin werden in 100 ml Tetrahydrofuran zum Sieden erhitzt und vorsichtig mit 13 g (0,1 Mol) Furan-2-carbonsäurechlorid versetzt. Man läßt 15 Minuten unter Rückfluß rühren und engt dann die Reaktionsmischung durch Abdestillieren des Lösungsmittels im Vakuum ein. Der Rückstand wird mit Methylenchlorid aufgenommen und mit Wasser gewaschen.

Man trennt die organische Phase ab, trocknet über Natriumsulfat und engt ein. Der Rückstand kristallisiert nach dem Verreiben mit Petrolether. Man erhält 22,5 g (89 % der Theorie) 2,6-Dimethyl-N-(2-furoyl)-N-propargyl-anilin vom Schmelzpunkt 109-112 °C.

Beispiel 4

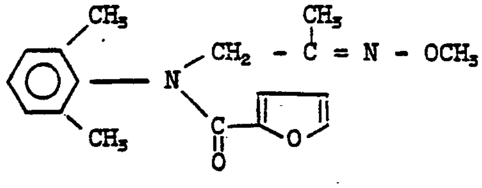

(Verfahren d)

7 g (0,024 Mol) 2,6-Dimethyl-N-(2-furoyl)-N-(2-hydroximino-prop-1-yl)-anilin (Beispiel 3) und 0,5 g Triethylbenzylammoniumchlorid werden in einem Zweiphasen-Gemisch aus 30 ml 50 %iger Natronlauge und 100 ml Methylenchlorid gelöst und unter heftigem Rühren mit 3,7 g (0,03 Mol) Dimethylsulfat versetzt. Die Temperatur steigt dabei von ca. 20 °C auf ca. 32 °C an. Nach 12-stündigem Rühren bei Raumtemperatur werden die Phasen getrennt. Die organische Phase wird nochmals mit Wasser gewaschen, über Natriumsulfat getrocknet und eingeengt. Das resultierende dunkle Oel wird in einer Kugelrohrapparatur destilliert (Siedepunkt 170 °C/13,33 PA). Das auskristallisierte Produkt (2,3 g) wird in Petrolether umkristallisiert. Man erhält 1,4 g (20 % der Theorie) 2,6-Dimethyl-N-(2-furoyl)-N-(2-methoximino-prop-1-yl)-anilin vom Schmelzpunkt 108-109 °C.

Entsprechend den Beispielen 1 bis 4 sowie entsprechend den Verfahrensvarianten (a) bis (f) werden die Verbindungen der allgemeinen Formel

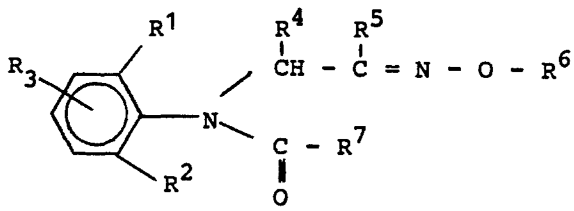

(I)

erhalten :

| Bsp. Nr. | $R^1$ | $R^2$ | $R^3$ | $R^4$ | $R^5$ | $R^6$ | $R^7$ | Physik. Konst. |
|---|---|---|---|---|---|---|---|---|
| 5 | $CH_3$ | $CH_3$ | H | H | $CH_3$ | H | $-CH_2OCH_3$ | Fp:12o-23°C |
| 6 | $CH_3$ | $CH_3$ | H | H | H | $CH_3$ | $-CH_2OCH_3$ | $n_D^{20}$:1,5261 |
| 7 | $CH_3$ | $CH_3$ | H | H | H | $CH_3$ | 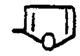 | Fp:85-91°C |
| 8 | $CH_3$ | $CH_3$ | H | $CH_3$ | $CH_3$ | $CH_3$ | $-CH_2OCH_3$ | $n_D^{20}$:1,5235 |
| 9 | $CH_3$ | $CH_3$ | H | $CH_3$ | H | $CH_3$ | 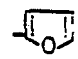 | $n_D^{20}$:1,5602 |
| 10 | $CH_3$ | $CH_3$ | H | $CH_3$ | $CH_3$ | $CH_3$ | 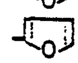 | $n_D^{20}$:1,5600 |
| 11 | $CH_2$ | $C_2H_5$ | H | H | H | $C_2H_5$ | $-CH_2OCH_3$ | $n_D^{20}$:1,5212 |
| 12 | $CH_3$ | $CH_3$ | H | $CH_3$ | $CH_3$ | $CH_3$ | $-CHCl_2$ | Fp:66-68°C |
| 13 | $CH_3$ | $CH_3$ | H | H | H | $C_2H_5$ | $-CH_2OCH_3$ | $n_D^{20}$:1,5210 |
| 14 | $CH_3$ | $CH_3$ | H | H | H | $C_3H_7$ | $-CH_2OCH_3$ | $n_D^{20}$:1,5173 |
| 15 | $CH_3$ | $CH_3$ | H | $CH_3$ | H | $C_2H_5$ | $-CHCl_2$ | $n_D^{20}$:1,5355 |
| 16 | $CH_3$ | $CH_3$ | H | $CH_3$ | H | $C_2H_5$ | $-CH_2OCH_3$ | $n_D^{20}$:1,5182 |
| 17 | $CH_3$ | $CH_3$ | H | $CH_3$ | H | $CH_3$ | $-COOCH_3$ | $n_D^{20}$:1,5188 |
| 18 | $CH_3$ | $CH_3$ | H | $CH_3$ | H | $CH_3$ | $-CHCl_2$ | $n_D^{20}$:1,5427 |
| 19 | $CH_3$ | $CH_3$ | H | $CH_3$ | H | $CH_3$ | 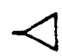 | $n_D^{20}$:1,5334 |
| 20 | $CH_3$ | $CH_3$ | H | $CH_3$ | H | $CH_3$ | $-CH_2-$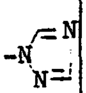 | Fp.: 81 - 104°C |
| 21 | $CH_3$ | $C_2H_5$ | H | $CH_3$ | H | $CH_3$ | $-CH_2OCH_3$ | $n_D^{20}$: 1,5231 |
| 22 | $CH_3$ | $C_2H_5$ | H | $CH_3$ | H | $C_2H_5$ | $-CH_2OCH_3$ | $n_D^{20}$: 1,5180 |
| 23 | $C_2H_5$ | $C_2H_5$ | H | $CH_3$ | H | $C_2H_5$ | $-CH_2OCH_3$ | $n_D^{20}$: 1,5172 |
| 24 | $CH_3$ | $CH_3$ | H | H | H | $CH_3$ | $-CHCl_2$ | Fp.: 73 - 78 °C |

(Siehe die Fortsetzung Seite 29)

(Fortsetzung)

| Bsp. Nr. | $R^1$ | $R^2$ | $R^3$ | $R^4$ | $R^5$ | $R^6$ | $R^7$ | Physik. Konst. |
|---|---|---|---|---|---|---|---|---|
| 25 | $CH_3$ | $CH_3$ | H | H | $CH_3$ | $-CH_2-CH=CH_2$ | $-CH_2OCH_3$ | Kp. $^{150°C}_{13,3322\ Pa}$ |
| 26 | $CH_3$ | $CH_3$ | H | H | $CH_3$ | $-CH_2-$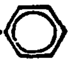 | $-CH_2OCH_3$ | Fp.: 60-62°C |
| 27 | $CH_3$ | $CH_3$ | H | H | H | $CH_3$ | $-CH_2-$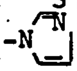 | Öl |
| 28 | $CH_3$ | $CH_3$ | H | H | H | $CH_3$ | $-COOCH_3$ | Kp.: $^{170°C}_{26.66\ Pa}$ |
| 29 | Cl | $CH_3$ | H | $CH_3$ | H | $CH_3$ | $-CH_2OCH_3$ | $n_D^{20}$: 1,5323 |
| 30 | $CH_3$ | $CH_3$ | H | H | H | $CH_3$ | $-CH_2-$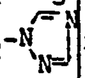 | $n_D^{20}$: 1,5528 |
| 31 | $CH_3$ | $CH_3$ | H | $CH_3$ | $CH_3$ | $CH_3$ | $-COOCH_3$ | $n_D^{20}$: 1,5195 |
| 32 | $CH_3$ | $CH_3$ | H | $CH_3$ | $CH_3$ | $CH_3$ | $-CH_2-$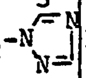 | Fp.: 101-08°C |
| 33 | $CH_3$ | $CH_3$ | H | $C_2H_5$ | H | $CH_3$ | $-CH_2OCH_3$ | $n_D^{20}$: 1,5221 |
| 34 | $CH_3$ | $CH_3$ | H | H | H | $CH_3$ | $-CH_2-OCH_2-C≡CH$ | Fp.: 85-87°C |
| 35 | $CH_3$ | $CH_3$ | H | H | $CH_3$ | 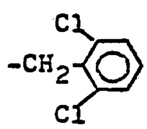 | $-CH_2OCH_3$ | Fp.: 72-74°C |
| 36 | $CH_3$ | $CH_3$ | H | H | H | $CH_3$ | $-CH=CH-CH_3$ | $n_D^{20}$: 1,5419 |
| 37 | $CH_3$ | $CH_3$ | H | $CH_3$ | H | $CH_3$ | $-CH=CH-CH_3$ | $n_D^{20}$: 1,5371 |
| 38 | $CH_3$ | $CH_3$ | H | H | H | $CH_3$ | $-CH=C(CH_3)_2$ | $n_D^{20}$: 1,5381 |
| 39 | $CH_3$ | $CH_3$ | H | $CH_3$ | H | $CH_3$ | $-CH_2-OCH_2-CH=CH_2$ | $n_D^{20}$: 1,5238 |
| 40 | $CH_3$ | $CH_3$ | H | $CH_3$ | H | $CH_3$ | $-CH_2OC_2H_5$ | $n_D^{20}$: 1,5170 |
| 41 | $CH_3$ | $CH_3$ | H | H | H | $CH_3$ | $-CH_2OC_2H_5$ | $n_D^{20}$: 1,5183 |
| 42 | $CH_3$ | $CH_3$ | H | $CH_3$ | H | $CH_3$ | $-CH_2-OCH_2-C≡CH$ | Öl |

## Ansprüche

1. N-Oximinoalkyl-anilide der allgemeinen Formel

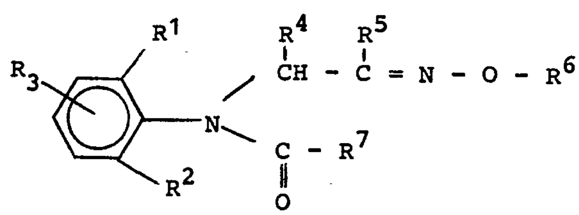

in welcher

$R^1$ für Wasserstoff, Alkyl oder Halogen steht,
$R^2$ für Wasserstoff oder Alkyl steht,
$R^3$ für Wasserstoff oder Alkyl steht,
$R^4$ für Wasserstoff oder Alkyl steht,
$R^5$ für Wasserstoff oder Alkyl steht,
$R^6$ für Wasserstoff, Alkyl, Alkenyl, Alkinyl, Alkoxyalkyl, Alkylthioalkyl oder gegebenenfalls durch Halogen, Alkyl, Halogenalkyl, Alkoxy, Alkylthio, Cyano und Nitro substituiertes Aralkyl steht und
$R^7$ für Furyl, Tetrahydrofuryl, Thiophenyl, Tetrahydrothiophenyl ; gegebenenfalls durch Alkyl substituiertes Isoxazolyl ; gegebenenfalls durch Cyano oder Thiocyano substituiertes Alkyl, Alkenyl und Alkinyl : Dihalogenalkyl, Cycloalkyl ; sowie die Gruppierungen $—CH_2Az$, $—CH_2—OR^8$, $—CH_2—SR^8$,

—$OR^8$, $SR^8$, —$CH_2$—$OSO_2R^8$, —$COOR^8$ und

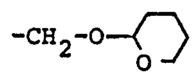

steht, wobei

$R^8$ für gegebenenfalls durch Halogen, Cyano und Thiocyano substituiertes Alkyl, Alkenyl, Alkinyl und Alkoxyalkyl steht, und

Az für Pyrazol-1-yl, 1,2,4-Triazol-1-yl und Imidazol-1-yl steht, ausgenommen die Verbindung in der
$R^1$ bis $R^4$ und $R^6$ für Wasserstoff,
$R^5$ für Methyl und
$R^7$ für Dichlormethyl stehen.

2. Verfahren zur Herstellung von N-Oximinoalkyl-aniliden der Formel I,

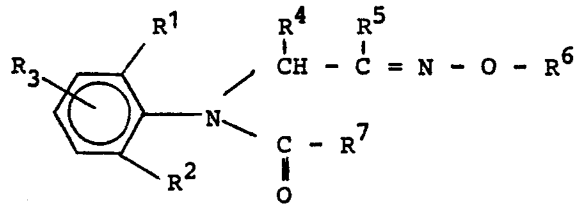

(I)

in welcher

$R^1$ für Wasserstoff, Alkyl oder Halogen steht,
$R^2$ für Wasserstoff oder Alkyl steht,
$R^3$ für Wasserstoff oder Alkyl steht,
$R^4$ für Wasserstoff oder Alkyl steht,
$R^5$ für Wasserstoff oder Alkyl steht,
$R^6$ für Wasserstoff, Alkyl, Alkenyl, Alkinyl, Alkoxyalkyl, Alkylthioalkyl oder gegebenenfalls durch Halogen, Alkyl, Halogenalkyl, Alkoxy, Alkylthio, Cyano und Nitro substituiertes Aralkyl steht und
$R^7$ für Furyl, Tetrahydrofuryl, Thiophenyl, Tetrahydrothiophenyl ; gegebenenfalls durch Alkyl substituiertes Isoxazolyl ; gegebenenfalls durch Cyano oder Thiocyano substituiertes Alkyl, Alkenyl und Alkinyl ; Dihalogenalkyl, Cycloalkyl ; sowie die Gruppierungen —$CH_2Az$, —$CH_2CR^8$, —$CH_2$—$SR^8$, —$OR^8$, —$SR^8$, —$CH_2$—$OSO_2R^8$, —$COOR^8$ und

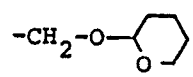

steht, wobei

$R^8$ für gegebenenfalls durch Halogen, Cyano und Thiocyano substituiertes Alkyl, Alkenyl, Alkinyl und Alkoxyalkyl steht, und

Az für Pyrazol-1-yl, 1,2,4-Triazol-1-yl und Imidazol-1-yl steht, ausgenommen die Verbindung in der
$R^1$, $R^2$, $R^3$, $R^4$ und $R^6$ für Wasserstoff,
$R^5$ für Methyl und
$R^6$ für Dichlormethyl stehen,

dadurch gekennzeichnet, daß man

a) Anilinoalkyloxim-ether der Formel

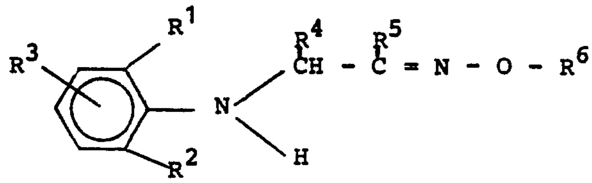

(II)

in welcher $R^1$, $R^2$, $R^3$, $R^4$, $R^5$ und $R^6$ die oben angegebene Bedeutung haben, mit Säurechloriden oder -bromiden bzw. -anhydriden der Formel

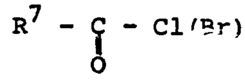

(IIIa)

bzw.

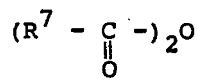

(IIIb)

in welchen $R^7$ die oben angegebene Bedeutung hat, in Gegenwart eines Verdünnungsmittels und gegebenenfalls in Gegenwart eines Säurebindemittels umsetzt, oder

b) Anilide der Formel

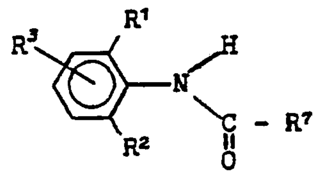

(IV)

in welcher $R^1$, $R^2$, $R^3$ und $R^7$ die oben angegebene Bedeutung haben, mit substituierten Oxim-ethern der Formel

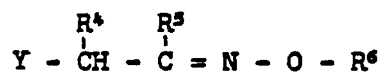

(V)

in welcher

$R^4$, $R^5$ und $R^6$ die oben angegebene Bedeutung haben und

Y für Halogen, den Mesylat- oder Tosylat-Rest steht,

in Gegenwart eines Säurebinders und gegebenenfalls in Gegenwart eines Verdünnungsmittels umsetzt, oder

c) N-substituierte Anilide der Formel

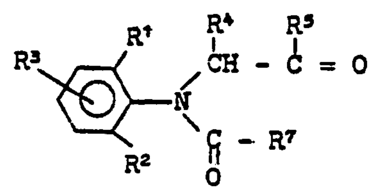

(VI)

in welcher $R^1$, $R^2$, $R^3$, $R^4$, $R^5$ und $R^7$ die oben angegebene Bedeutung haben mit Salzen von Hydroxylamin-(Derivaten) der Formel

$$H_2N—O—R^6 \qquad (VII)$$

in welcher $R^6$ die oben angegebene Bedeutung hat, in Gegenwart eines Verdünnungsmittels und in Gegenwart eines Säurebindemittels umsetzt, oder

d) Alkalisalze von Oximen der Formel

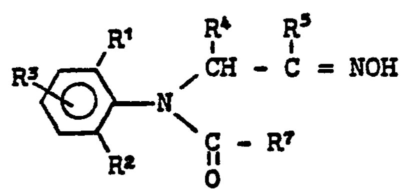

(VIII)

in welcher $R^1$, $R^2$, $R^3$, $R^4$, $R^5$ und $R^7$ die oben angegebene Bedeutung haben, mit Verbindungen der Formel

$$X—R^9 \qquad (IX)$$

in welcher

$R^9$ für Alkyl, Alkenyl, Alkinyl, Alkoxyalkyl, Alkylthioalkyl oder gegebenenfalls durch Halogen, Alkyl, Halogenalkyl, Alkoxy, Alkylthio, Cyano und Nitro substituiertes Aralkyl steht und

X für Chlor, Brom, Mesyl, Tosyl oder Methoxysulfonyloxy steht,

in Gegenwart eines organischen Verdünnungsmittels oder in Gegenwart eines organisch-wässrigen Zweiphasen-systems in Gegenwart eines Phasentransferkatalysators umsetzt, wobei die Alkalisalze der Oxime der Formel (VIII) in situ erzeugt werden, oder

e) Halogenacetanilide der Formel

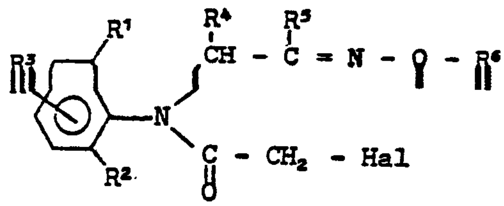

(X)

in welcher

$R^1$, $R^2$, $R^3$, $R^4$, $R^5$ und $R^6$ die oben angegebene Bedeutung haben und

Hal für Chlor, Brom oder Jod steht,

mit Verbindungen der Formel

$$B{-}R^{10} \qquad (XI)$$

in welcher

B für Wasserstoff oder ein Alkalimetall steht und

$R^{10}$ für Az und die Gruppierungen $-OR^8$ oder $-SR^8$ steht,

in Gegenwart eines Verdünnungsmittels und gegebenenfalls in Gegenwart eines Säurebinders umsetzt, oder

f) Hydroxyacetanilide der Formel

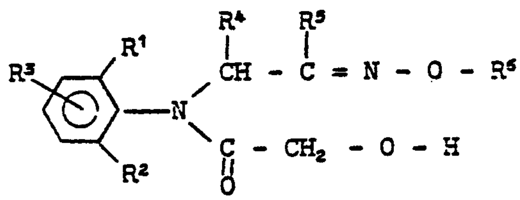

(XII)

in welcher $R^1$, $R^2$, $R^3$, $R^4$, $R^5$ und $R^6$ die oben angegebene Bedeutung haben,

(1) gegebenenfalls nach Aktivierung mittels Alkalimetall mit Halogeniden der Formel

$$Hal{-}R^{11} \qquad (XIII)$$

in welcher

Hal die oben angegebene Bedeutung hat und

$R^{11}$ für den Rest $R^8$ sowie die Gruppierung $-SO_2R^8$ steht, wobei $R^8$ die oben angegebene Bedeutung hat,

in Gegenwart eines Verdünnungsmittels und gegebenenfalls in Gegenwart eines Säurebinders umsetzt, oder

(2) mit Dihydropyran der Formel

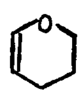

(XIV)

in Gegenwart eines Verdünnungsmittels und gegebenenfalls in Gegenwart eines Katalysators umsetzt.

3. Fungizide Mittel, gekennzeichnet durch einen Gehalt an mindestens einem N-Oxyminoalkyl-anilid der Formel IA.

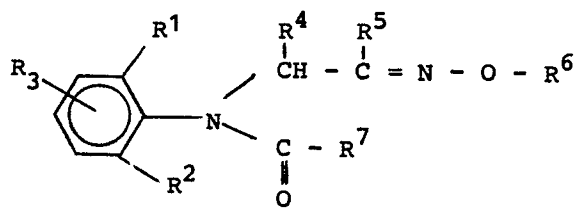

(IA)

in welcher

$R^1$ für Wasserstoff, Alkyl oder Halogen steht,

$R^2$ für Wasserstoff oder Alkyl steht,

$R^3$ für Wasserstoff oder Alkyl steht,

$R^4$ für Wasserstoff oder Alkyl steht,

$R^5$ für Wasserstoff oder Alkyl steht,

$R^6$ für Wasserstoff, Alkyl, Alkenyl, Alkinyl,Alkoxyalkyl, Alkylthioalkyl oder gegebenenfalls durch Halogen, Alkyl, Halogenalkyl, Alkoxy, Alkylthio, Cyano und Nitro substituiertes Aralkyl steht und

$R^7$ für Furyl, Tetrahydrofuryl, Thiophenyl, Tetrahydrothiophenyl ; gegebenenfalls durch Alkyl substituiertes Isoxazolyl; gegebenenfalls durch Cyano oder Thiocyano substituiertes Alkyl, Alkenyl und Alkinyl; Dihalogenalkyl, Cycloalkyl; sowie die Gruppierungen $-CH_2Az$, $-CH_2-OR^8$, $-CH_2-SR^8$, $-OR^8$, $SR^8$, $-CH_2-OSO_2R^8$, $-COOR^8$ und

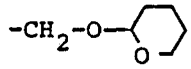

steht, wobei

$R^8$ für gegebenenfalls durch Halogen, Cyano und Thiocyano substituiertes Alkyl, Alkenyl, Alkinyl und Alkoxyalkyl steht, und

Az für Pyrazol-1-yl, 1,2,4-Triazol-1-yl und Imidazol-1-yl steht.

4. Verfahren zur Bekämpfung von Pilzen, dadurch gekennzeichnet, daß man N-Oximinoalkyl-anilide der Formel IA auf Pilze oder ihren Lebensraum einwirken läßt.

5. Verwendung von N-Oximinoalkyl-anilide der Formel IA zur Bekämpfung von Pilzen.

6. Verfahren zur Herstellung von fungiziden Mitteln, dadurch gekennzeichnet, daß man N-Oximinoalkyl-anilide der Formel IA mit Streckmitteln und/oder oberflächenaktiven Mittel vermischt.

**Claims**

1. N-Oximinoalkyl-anilides of the general formula

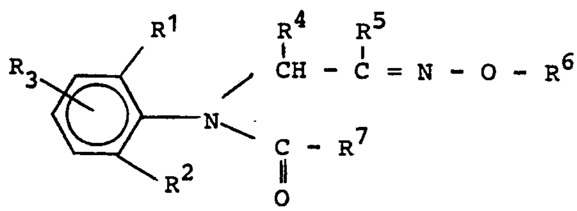

(I)

in which

$R^1$ represents hydrogen, alkyl or halogen,

$R^2$ represents hydrogen or alkyl,

$R^3$ represents hydrogen or alkyl,

$R^4$ represents hydrogen or alkyl,

$R^5$ represents hydrogen or alkyl,

$R^6$ represents hydrogen, alkyl, alkenyl, alkinyl, alkoxylalkyl, alkylthioalkyl or aralkyl which is optionally substituted by halogen, alkyl, halogenoalkyl, alkoxy, alkylthio, cyano or nitro, and

$R^7$ represents furyl, tetrahydrofuryl, thiophenyl, or tetrahydrothiophenyl; isoxazolyl which is optionally substituted by alkyl; alkyl, alkenyl or alkinyl, optionally substituted by cyano or thiocyano; dihalogenoalkyl or cycloalkyl; or the grouping $-CH_2Az$, $-CH_2-OR^8$, $-CH_2-SR^8$, $-OR^8$, $SR^8$, $-CH_2-OSO_2R^8$, $-COOR^8$ or

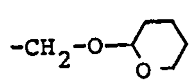

wherein

$R^8$ represents alkyl, alkenyl, alkinyl or alkoxyalkyl, optionally substituted by halogen, cyano or thiocyano, and

Az represents pyrazol-1-yl, 1,2,4-triazol-1-yl or imidazol-1-yl, with the exception of the compound in which

$R^1$ to $R^4$ and $R^6$ represent hydrogen,

$R^5$ represents methyl and

$R^7$ represents dichloromethyl.

2. Process for the preparation of N-oximinoalkyl-anilides of the formula I

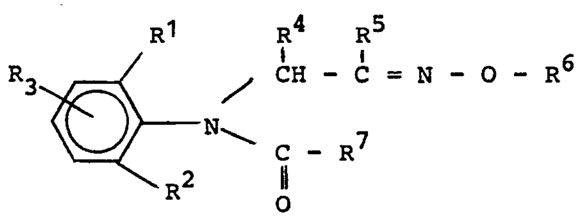

(I)

in which

$R^1$ represents hydrogen, alkyl or halogen,

$R^2$ represents hydrogen or alkyl,

$R^3$ represents hydrogen or alkyl,

$R^4$ represents hydrogen or alkyl,

$R^5$ represents hydrogen or alkyl,

$R^6$ represents hydrogen, alkyl, alkenyl, alkinyl, alkoxylalkyl, alkylthioalkyl or aralkyl which is optionally substituted by halogen, alkyl, halogenoalkyl, alkoxy, alkylthio, cyano or nitro, and

$R^7$ represents furyl, tetrahydrofuryl, thiophenyl, or tetrahydrothiophenyl ; isoxazolyl which is optionally substituted by alkyl ; alkyl, alkenyl or alkinyl, optionally substituted by cyano or thiocyano ; dihalogenoalkyl or cycloalkyl ; or the grouping $-CH_2Az$, $-CH_2-OR^8$, $-CH_2-SR^8$, $-OR^8$, $SR^8$, $-CH_2-OSO_2R^8$, $-COOR^8$ or

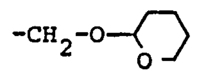

wherein

$R^8$ represents alkyl, alkenyl, alkinyl or alkoxyalkyl, optionally substituted by halogen, cyano or thiocyano, and

Az represents pyrazol-1-yl, 1,2,4-triazol-1-yl or imidazol-1-yl, with the exception of the compound in which

$R^1$, $R^2$, $R^3$, $R^4$ and $R^6$ represent hydrogen,

$R^5$ represents methyl and

$R^6$ represents dichloromethyl,

characterised in that

a) anilinoalkyl oxime ethers of the formula

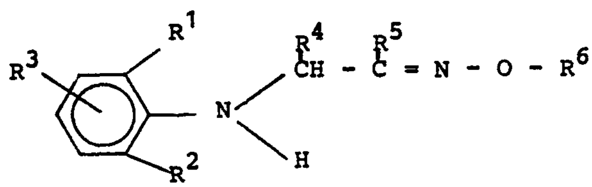 (II)

in which $R^1$, $R^2$, $R^3$, $R^4$, $R^5$ and $R^6$ have the meaning indicated above,
are reacted with acid chlorides or bromides or anhydrides of the formula

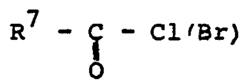 (IIIa)

or

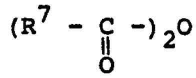 (IIIb)

in which $R^7$ has the meaning indicated above,
in the presence of a diluent and if appropriate in the presence of an acid-binding agent, or

b) anilides of the formula

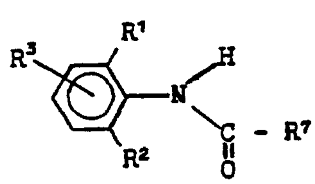 (IV)

in which $R^1$, $R^2$, $R^3$ and $R^7$ have the meaning indicated above,
are reacted with substituted oxime ethers of the formula

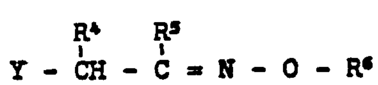 (V)

in which

$R^4$, $R^5$ und $R^6$ have the meaning indicated above and
Y represents halogen or the mesylate or tosylate radical,
in the presence of an acid-binding agent and if appropriate in the presence of a diluent, or
c) N-substituted anilides of the formula

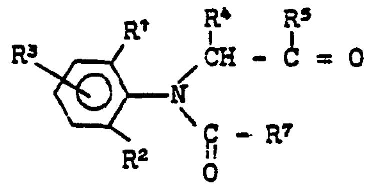

(VI)

in which $R^1$, $R^2$, $R^3$, $R^4$, $R^5$ and $R^7$ have the meaning indicated above, are reacted with salts of hydroxylamine (derivatives) of the formula

$$H_2N—O—R^6 \qquad (VII)$$

in which $R^6$ has the meaning indicated above, in the presence of a diluent and in the presence of an acid-binding agent, or
d) alkali metal salts of oximes of the formula

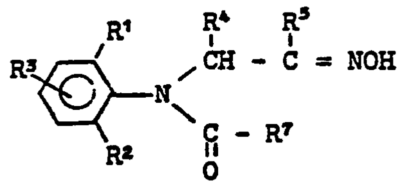

(VIII)

in which $R^1$, $R^2$, $R^3$, $R^4$, $R^5$ and $R^7$ have the meaning indicated above, are reacted with compounds of the formula

$$X—R^9 \qquad (IX)$$

in which
$R^9$ represents alkyl, alkenyl, alkinyl, alkoxyalkyl, alkylthioalkyl or aralkyl which is optionally substituted by halogen, alkyl, halogenoalkyl, alkoxy, alkylthio, cyano or nitro and
X represents chlorine, bromine, mesyl, tosyl or methoxysulphonyloxy,
in the presence of an organic diluent or in the presence of an organic-aqueous two-phase system in the presence of a phase transfer catalyst, the alkali metal salts of the oximes of the formula (VIII) being produced in situ, or
e) halogenoacetanilides of the formula

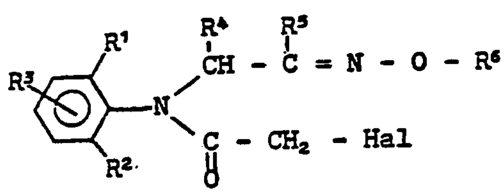

(X)

in which
$R^1$, $R^2$, $R^3$, $R^4$, $R^5$ and $R^6$ have the meaning indicated above and
Hal represents chlorine, bromine or iodine,
are reacted with compounds of the formula

$$B—R^{10} \qquad (XI)$$

in which
B represents hydrogen or an alkali metal and
$R^{10}$ represents Az or the grouping $—OR^8$ or $—SR^8$,
in the presence of a diluent and if appropriate in the presence of an acid-binding agent, or
f) hydroxyacetanilides of the formula

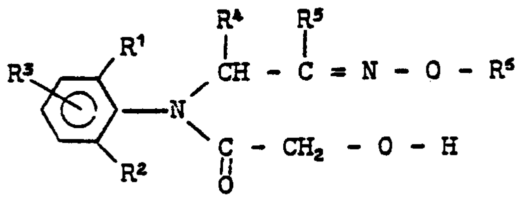

(XII)

in which
$R^1$, $R^2$, $R^3$, $R^4$, $R^5$ and $R^6$ have the meaning indicated above,

(1) are reacted, if appropriate after activation by means of an alkali metal, with halides of the formula

$$Hal{-}R^{11} \qquad (XIII)$$

in which

Hal has the meaning indicated above and

$R^{11}$ represents the radical $R^8$ or the grouping $-SO_2R^8$, wherein

$R^8$ has the meaning indicated above,

in the presence of a diluent and if appropriate in the presence of an acid-binding agent, or

(2) are reacted with dihydropyrane of the formula

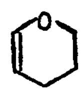

(XIV)

in the presence of a diluent and if appropriate in the presence of a catalyst.

3. Fungicidal agents, characterised in that they contain at least one N-oxyminoalkyl-anilide of the formula IA

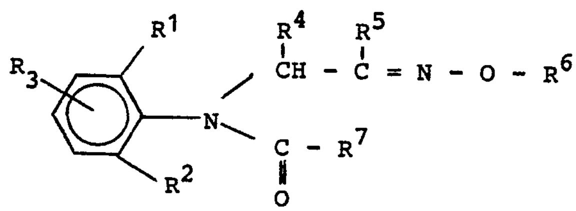

in which

$R^1$ represents hydrogen, alkyl or halogen,

$R^2$ represents hydrogen or alkyl,

$R^3$ represents hydrogen or alkyl,

$R^4$ represents hydrogen or alkyl,

$R^5$ represents hydrogen or alkyl,

$R^6$ represents hydrogen, alkyl, alkenyl, alkinyl, alkoxylalkyl, alkylthioalkyl or aralkyl which is optionally substituted by halogen, alkyl, halogenoalkyl, alkoxy, alkylthio, cyano or nitro, and

$R^7$ represents furyl, tetrahydrofuryl, thiophenyl, or tetrahydrothiophenyl ; isoxazolyl which is optionally substituted by alkyl ; alkyl, alkenyl or alkinyl, optionally substituted by cyano or thiocyano ; dihalogenoalkyl or cycloalkyl ; or the grouping $-CH_2Az$, $-CH_2-OR^8$, $-CH_2-SR^8$, $-OR^8$, $SR^8$, $-CH_2-OSO_2R^8$, $-COOR^8$ or

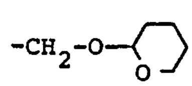

wherein

$R^6$ represents alkyl, alkenyl, alkinyl or alkoxyalkyl, optionally substituted by halogen, cyano or thiocyano, and

Az represents pyrazol-1-yl, 1,2,4-triazol-1-yl or imidazol-1-yl.

4. Process for combating fungi, characterised in that N-oximinoalkyl-anilides of the formula IA are allowed to act on fungi or their environment.

5. Use of N-oximinoalkyl-anilides of the formula IA for combating fungi.

6. Process for the preparation of fungicidal agents, characterised in that N-oximinoalkyl-anilides of the formula IA are mixed with extenders and/or surface-active agents.

**Revendications**

1. N-oximinoalkyl-anilides de formule générale

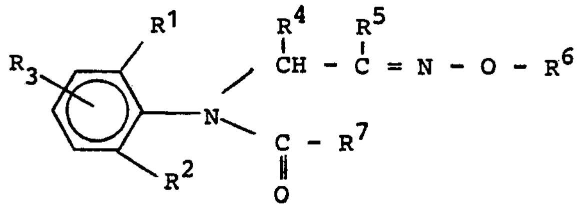

(I)

dans laquelle

$R^1$ est l'hydrogène, un groupe alkyle ou un halogène,

$R^2$ est l'hydrogène ou un groupe alkyle,

$R^3$ est l'hydrogène ou un groupe alkyle,

$R^4$ est l'hydrogène ou un groupe alkyle,

$R^5$ est l'hydrogène ou un groupe alkyle,

$R^6$ est l'hydrogène, un groupe alkyle, alcényle, alcynyle, alkoxyalkyle, alkylthioalkyle ou un groupe aralkyle éventuellement substitué par un halogène ou un radical alkyle, halogénalkyle, alkoxy, alkylthio, cyano et nitro, et

$R^7$ est un groupe furyle, tétrahydrofuryle, thiophényle, tétrahydrothiophényle ; isoxazolyle éventuellement substitué par un radical alkyle ; un groupe alkyle, alcényle ou alcynyle éventuellement substitué par un radical cyano ou thiocyano, un groupe dihalogénalkyle, cycloalkyle ; ainsi que les groupements $—CH_2Az$, $—CH_2—OR^8$, $—CH_2—SR^8$, $—OR^8$, $—SR^8$, $—CH_2—OSO_2R^8$, $—COOR^8$ et

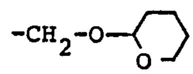

$R^8$ représentant un groupe alkyle, alcényle, alcynyle ou alkoxyalkyle éventuellement substitué par un halogène ou un radical cyano ou thiocyano, et

Az représentant un groupe pyrazole-1-yle, 1,2,4-triazole-1-yle ou imidazole-1-yle, excepté le composé dans lequel

$R^1$ à $R^4$ et $R^6$ représentent l'hydrogène,

$R^5$ est un groupe méthyle et

$R^7$ est un groupe dichlorométhyle.

2. Procédé de production de N-oximinoalkylanilides de formule I

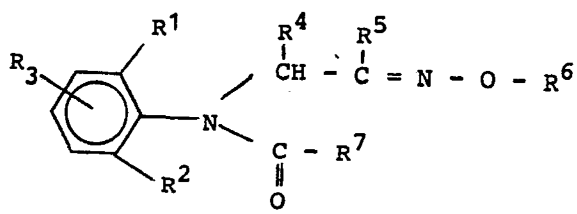

(I)

dans laquelle

$R^1$ est l'hydrogène, un groupe alkyle ou un halogène,

$R^2$ est l'hydrogène ou un groupe alkyle,

$R^3$ est l'hydrogène ou un groupe alkyle,

$R^4$ est l'hydrogène ou un groupe alkyle,

$R^5$ est l'hydrogène ou un groupe alkyle,

$R^6$ est l'hydrogène, un groupe alkyle, alcényle, alcynyle, alkoxyalkyle, alkylthioalkyle ou un groupe aralkyle éventuellement substitué par un halogène ou un radical alkyle, halogénalkyle, alkoxy, alkylthio, cyano et nitro, et

$R^7$ est un groupe furyle, tétrahydrofuryle, thiophényle, tétrahydrothiophényle ; isoxazolyle éventuellement substitué par un radical alkyle ; un groupe alkyle, alcényle ou alcynyle éventuellement substitué par un radical cyano ou thiocyano, un groupe dihalogénalkyle, cycloalkyle ; ainsi que les groupements $—CH_2Az$, $—CH_2OR^8$, $—CH_2—SR^8$, $—OR^8$, $—SR^8$, $—CH_2—OSO_2R^8$, $—COOR^8$ et

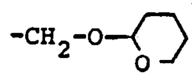

$R^8$ représentant un groupe alkyle, alcényle, alcynyle ou alkoxyalkyle éventuellement substitué par un halogène ou un radical cyano ou thiocyano, et

Az représentant un groupe pyrazole-1-yle, 1,2,4-triazole-1-yle ou imidazole-1-yle, excepté le composé dans lequel

$R^1$ à $R^4$ et $R^6$ représentent l'hydrogène,
$R^5$ est un groupe méthyle et
$R^6$ est un groupe dichlorométhyle,

caractérisé en ce que

a) on fait réagir un éther d'anilinoalkyloxime de formule

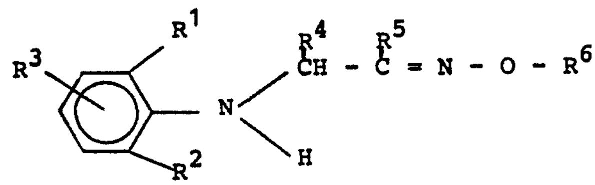

(II)

dans laquelle $R^1$, $R^2$, $R^3$, $R^4$, $R^5$ et $R^6$ ont la définition indiquée ci-dessus, avec des chlorures ou bromures et des anhydrides d'acides de formules

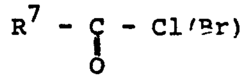

(IIIa)

et

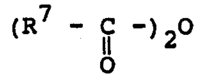

(IIIb)

dans lesquelles $R^7$ à la définition indiquée ci-dessus, en présence d'un diluant et, le cas échéant, en présence d'un accepteur d'acide, ou

b) on fait réagir des anilides de formule

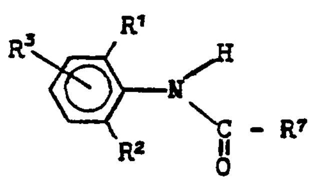

(IV)

dans laquelle $R^1$, $R^2$, $R^3$ et $R^7$ ont la définition indiquée ci-dessus, avec des éthers d'oximes substitués de formule

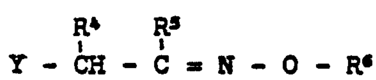

(V)

dans laquelle

$R^4$, $R^5$ et $R^6$ ont la définition indiquée ci-dessus et
Y représente un halogène, le reste mésylate ou le reste tosylate,

en présence d'un accepteur d'acide et, le cas échéant, en présence d'un diluant, ou

c) on fait réagir des anilides N-substitués de formule

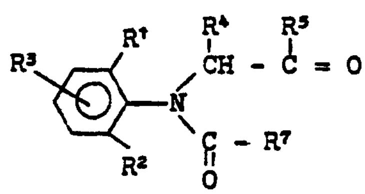

(VI)

dans laquelle $R^1$, $R^2$, $R^3$, $R^4$, $R^5$ et $R^7$ ont la définition indiquée ci-dessus avec des sels (de dérivés) de l'hydroxylamine de formule

$$H_2N—O—R^6 \qquad (VII)$$

dans laquelle $R^6$ a la définition indiquée ci-dessus,

en présence d'un diluant et en présence d'un accepteur d'acide, ou

d) on fait réagir des sels alcalins d'oximes de formule

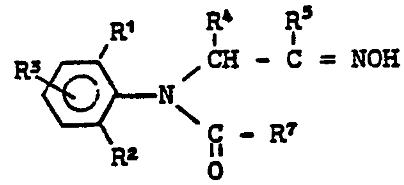

(VIII)

dans laquelle $R^1$, $R^2$, $R^3$, $R^4$, $R^5$ et $R^7$ ont la définition indiquée ci-dessus, avec des composés de formule

$$X—R^9 \qquad (IX)$$

dans laquelle

$R^9$ est un groupe alkyle, alcényle, alcynyle, alkoxy-alkyle, alkylthioalkyle ou un groupe aralkyle éventuellement substitué par un halogène ou un radical alkyle, halogénalkyle, alkoxy, alkylthio, cyano ou nitro et

X représente le chlore, le brome, le groupe mésyle, tosyle ou méthoxysulfonyloxy,

en présence d'un diluant organique ou en présence d'un système hydro-organique de deux phases en présence d'un catalyseur de transfert de phases, les sels alcalins des oximes de formule (VIII) étant produits *in situ*, ou

e) on fait réagir des halogénacétanilides de formule

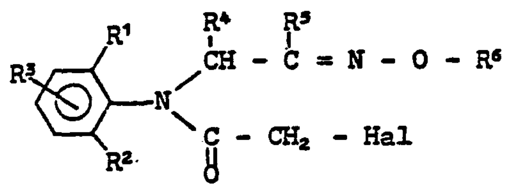

(X)

dans laquelle

$R^1$, $R^2$, $R^3$, $R^4$, $R^5$ et $R^6$ ont la définition indiquée ci-dessus et

Hal désigne le chlore, le brome ou l'iode,

avec des composés de formule

$$B—R^{10} \qquad (XI)$$

dans laquelle

B désigne l'hydrogène ou un métal alcalin et

$R^{10}$ représente Az et les groupements $—OR^8$ ou $—SR^8$,

en présence d'un diluant et, le cas échéant en présence d'un accepteur d'acide, ou

f) on fait réagir des hydroxyacétanilides de formule

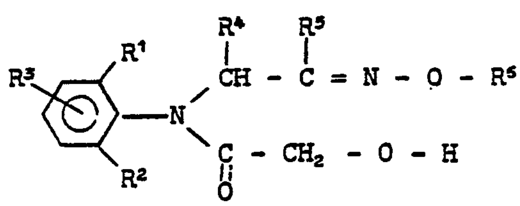

(XII)

dans laquelle $R^1$, $R^2$, $R^3$, $R^4$, $R^5$ et $R^6$ ont la définition indiquée ci-dessus,

(1) éventuellement après activation au moyen d'un métal alcalin, avec des halogénures de formule

$$Hal—R^{11} \qquad (XIII)$$

dans laquelle

Hal a la définition indiquée ci-dessus et

$R^{11}$ représente le reste $R^8$ de même que le groupement $—SO_2R^8$,

$R^8$ ayant la définition indiquée ci-dessus,

en présence d'un diluant et, le cas échéant en présence d'un accepteur d'acide, ou bien

(2) avec le dihydropyranne de formule

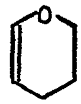

(XIV)

en présence d'un diluant et, le cas échéant en présence d'un catalyseur.

3. Compositions fongicides, caractérisées par une teneur en au moins un N-oximinoalkyl-anilide de formule IA

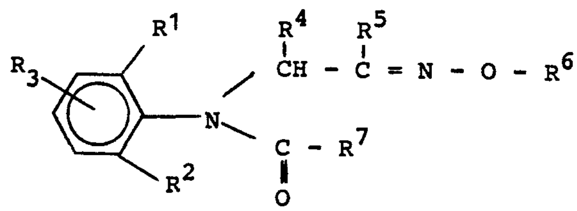

(I)

dans laquelle

$R^1$ est l'hydrogène, un groupe alkyle ou un halogène,

$R^2$ est l'hydrogène ou un groupe alkyle,

$R^3$ est l'hydrogène ou un groupe alkyle,

$R^4$ est l'hydrogène ou un groupe alkyle,

$R^5$ est l'hydrogène ou un groupe alkyle,

$R^6$ est l'hydrogène ou un groupe alkyle, alcényle, alcynyle, alkoxyalkyle, alkylthioalkyle ou un groupe aralkyle éventuellement substitué par un halogène ou un radical alkyle, halogénalkyle, alkoxy, alkylthio, cyano ou nitro et

$R^7$ est un groupe furyle, tétrahydrofuryle, thiophényle, tétrahydrothiophényle ; un groupe isoxazolyle éventuellement substitué par un radical alkyle ; un groupe alkyle, alcényle ou alcynyle éventuellement substitué par un radical cyano ou thiocyano ; un groupe dihalogénalkyle, cycloalkyle ; de même que les groupements $—CH_2Az$, $—CH_2—OR^8$, $—CH_2—SR^8$, $—OR^8$, $—SR^8$, $—CH_2—OSO_2R^8$, $—COOR^8$ et

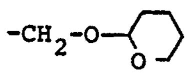

$R^8$ désignant un groupe alkyle, alcényle, alcynyle ou alkoxyalkyle éventuellement substitué par un halogène ou un radical cyano ou thiocyano, et

Az représentant un groupe pyrazole-1-yle, 1,2,4-triazole-1-yle et imidazole-1-yle.

4. Procédé de lutte contre des champignons, caractérisé en ce qu'on fait agir des N-oximinoalkyl-anilides de formule IA sur les champignons ou leur milieu.

5. Utilisation de N-oximinoalkyl-anilides de formule IA pour la lutte contre des champignons.

6. Procédé de production de compositions fongicides, caractérisé en ce qu'on mélange des N-oximino-alkyl-anilides de formule IA avec des diluants et/ou des agents tensio-actifs.